(19) Europäisches Patentamt / European Patent Office / Office européen des brevets

(11) **EP 1 756 774 B1**

(12) **FASCICULE DE BREVET EUROPEEN**

(45) Date de publication et mention
de la délivrance du brevet:
**13.11.2013 Bulletin 2013/46**

(21) Numéro de dépôt: **05777257.6**

(22) Date de dépôt: **10.06.2005**

(51) Int Cl.:
***G06T 11/00*** *(2006.01)*

(86) Numéro de dépôt international:
**PCT/FR2005/001437**

(87) Numéro de publication internationale:
**WO 2006/003312 (12.01.2006 Gazette 2006/02)**

(54) **PROCEDE DESTINE A REDUIRE L'EXPOSITION DES RADIATIONS , DES FAISCEAUX A LUMIERE INFRAROUGE ,ULTRASONIQUES OU DES IMPULSIONS MAGNETIQUES DANS DES DISPOSOTIFS D'IMAGERIE MEDICALE**

VERFAHREN ZUR VERRINGERUNG DER BELICHTUNG MIT INFRAROTLICHTSTRAHLEN, ULTRASCHALL- ODER MAGNETISCHEN IMPULSSTRAHLEN VON MEDIZINISCHEN BILDGEBUNGSVORRICHTUNGEN

METHOD FOR REDUCING EXPOSURE TO INFRARED LIGHT BEAM, ULTRASOUND OR MAGNETIC IMPULSE RAYS IN MEDICAL IMAGING DEVICES

(84) Etats contractants désignés:
**AT BE BG CH CY CZ DE DK EE ES FI FR GB GR HU IE IS IT LI LT LU MC NL PL PT RO SE SI SK TR**

(30) Priorité: **16.06.2004 FR 0406497**
**18.11.2004 FR 0452677**

(43) Date de publication de la demande:
**28.02.2007 Bulletin 2007/09**

(73) Titulaire: **Beyrard, Norbert**
**01220 Divonne-Les-Bains (FR)**

(72) Inventeur: **Beyrard, Norbert**
**01220 Divonne-Les-Bains (FR)**

(74) Mandataire: **Gaglione, Renaud et al**
**240, rue du Quart**
**01710 Thoiry (FR)**

(56) Documents cités:
**EP-A- 0 964 366     EP-A- 1 096 428**
**US-A- 5 241 471     US-A- 5 442 674**

- **JIANG M ET AL: "CONVERGENCE STUDIES ON ITERATIVE ALGORITHMS FOR IMAGE RECONSTRUCTION" IEEE TRANSACTIONS ON MEDICAL IMAGING, IEEE INC. NEW YORK, US, vol. 22, no. 5, mai 2003 (2003-05), pages 569-579, XP001164801 ISSN: 0278-0062**
- **GORDON R: "A TUTORIAL ON ART" IEEE TRANSACTIONS ON NUCLEAR SCIENCE, IEEE INC. NEW YORK, US, vol. NS-21, no. 3, juin 1974 (1974-06), pages 78-93, XP000972999 ISSN: 0018-9499**
- **HERMAN G T ET AL: "ALGEBRAIC RECONSTRUCTION TECHNIQUES CAN BE MADE COMPUTATIONALLY EFFICIENT" IEEE TRANSACTIONS ON MEDICAL IMAGING, IEEE INC. NEW YORK, US, vol. 12, no. 3, 1 septembre 1993 (1993-09-01), pages 600-609, XP000412338 ISSN: 0278-0062**

## Description

L'invention est relative à un procédé destiné à réduire l'exposition à des radiations, des faisceaux ultrasoniques à lumière infrarouge, ou des impulsions magnétiques dans un organisme, dans des dispositifs d'imagerie médicale, et comprend une méthode de traitement d'un signal ou d'un ensemble de signaux en vue de produire dans des conditions très améliorée des images numériques de l'intérieur d'un organisme, pour détecter et traiter le cas échéants les anomalies ou les singularités de l'organisme examiné.

Différentes méthodes sont maintenant couramment utilisées dans ce but, en particulier, la radiographie, la scannographie, l'échographie, la résonance magnétique pour obtenir et traiter des informations concernant un ensemble de points ou de zones élémentaires, situés dans un organisme, en vue de produire des éléments d'information relatifs à chaque point, et susceptibles d'être exprimés par des images utilisables par des praticiens à la recherche d'anomalies ou de singularités.

De manière classique, on obtient par un calcul approprié, une valeur, associée à chaque point ou zone du coefficient d'atténuation dans le cas du scanner médical, de réflexion dans le cas de l'échographie, ou de la densité de protons dans le cas de la résonance magnétique pour produire une image interne de l'organisme.

L'exemple du scanner médical illustre le niveau de l'art en la matière.

L'imagerie médicale est en effet un moyen fondamental pour détecter et traiter le cancer, et un certain nombre de maladies graves, ou d'observer les organes internes d'un patient. Mais il est apparut nécessaire à la communauté médicale, d'aller beaucoup plus loin en exprimant la demande de mise au point d'un procédé d'imagerie médicale, à haute définition à partir d'une amélioration significative d'obtention et de traitement des signaux et des images qu'ils expriment.

Il faut d'abord rappeler, à titre d'exemple, les principes de la scannographie et l'état actuel de l'art en la matière.

La Scannographie (ou Tomodensitométrie), a été découverte par un ingénieur de la firme EMI, G. N. Hounsfield, en 1968.

Le brevet (US 3 924 131, US 3 919 552) de 1972 s'intitule : *"A method and apparatus for examination of a body by radiation such as X or gamma-radiation"*.

Cette invention valut en 1979 le prix NOBEL à son inventeur.

Le principe est le suivant :

Un faisceau de rayons X, balaye un plan défini, il traverse un organe de manière linéaire et frappe une plaque ou un détecteur radiographique. La traversée de l'organe provoque une atténuation du faisceau, dont la mesure peut être effectuée grâce au détecteur. Le balayage de manière croisée, dans le plan de coupe, produit une série d'informations traitée par des logiciels appropriés sur un ordinateur associé.

En effet suivant chaque axe de balayage dans un milieu hétérogène l'atténuation peut s'exprimer par une loi exponentielle, tenant compte de l'absorption photoélectrique et de la diffusion par effet Compton.

Soit 10, la valeur de référence

1 x la valeur en un point X,

On peut écrire la relation suivante :

$$\int A(x)\,dx = F$$

$$I = Io\, e^{-F}$$

$$ln = Io\, E$$

$$Ln = \frac{Io}{Ik} = \int_0^t A(x)\,dx$$

Dont on déduit par discrétisation :

$$\text{Ln } \frac{Io}{In} = \int_{0}^{t} A \ (x) \ dx = A_1 X_1 + A_2 X_2 + \ldots\ldots + A_n X_n$$

Les valeurs successives, Ai, $A_2$, ........., $A_n$ correspondent aux valeurs de chaque segment définis par $X_1$, $X_2$, ......, $X_n$.

On peut alors exprimer par une série d'équations, les profils de chaque balayage associé à un angle (ou à une position) précis.

On peut définir une échelle particulière par la valeur, relative à une valeur de référence du coefficient d'atténuation, celle de l'eau par exemple, ou toute autre molécule convenablement choisie.

L'échelle, utilisée le plus souvent est celle relative à une molécule, abondante dans tous les organismes vivants, l'eau.

Si l'on appelle A (h2o), le coefficient d'atténuation de l'eau, on peut utiliser une échelle relative telle que :

$$Bn = [An - A(h2o)] * 1000 \ / \ A(h2o)$$

La valeur du coefficient de l'eau peut être définie comme égale à 1 ou à 0, créant ainsi un système de notation facile à utiliser puisque l'eau est un composant essentiel du corps humain.

D'autres systèmes peuvent d'ailleurs être utilisées, en fonction de la manière dont l'information obtenue sera exprimée (de manière visuelle). On choisit souvent la valeur de 1000 pour l'os et de -1000 pour l'air.

Le traitement informatique d'un nombre suffisant de balayages croisés, définissant en fait des petites cellules ou zones élémentaires, permet la résolution d'un ensemble d'équations linéaires à condition que le nombre de balayages soit égal aux nombre de cellules.

L'édition et l'utilisation de l'information sont effectuées par un ordinateur associé.

L'ordinateur collecte l'ensemble des données et calcule ainsi la valeur du coefficient d'atténuation de chaque zone élémentaire.

L'information résultant des calculs est traduite par une carte du plan de coupe Tomographique.

L'ensemble des cartes constitue l'image scanner en trois dimensions de l'analyse, ce qui autorise des coupes longitudinales ou transversales.

L'interprétation médicale s'appuie ainsi sur une véritable image intérieure des tissus.

De telles images permettent de vérifier l'état de certains os, aussi bien que l'état du cerveau, pour détecter tumeur ou autre anomalie.

Les explorations sont précédées ou complétées par d'autres explorations par exemple échographie ultrasonore ou imagerie par résonance magnétique.

Le scanner et les méthodes qu'il a initiées restent un outil essentiel de l'exploration médicale.

A l'origine on procédait à une série de déplacements angulaires de l'ordre de 3 degrés, répétés une centaine de fois.

Les perfectionnements apportés depuis permettent d'associer plusieurs faisceaux à des barrettes de détection d'une longueur suffisante pour multiplier le nombre de mesures effectuées au même instant grâce à des détecteurs multiples.

Dans les scanners de cinquième génération on utilise des barrettes de détecteur perpendiculaires aux plans de coupe pour éviter tout déplacement.

L'image obtenue résulte d'un processus par étapes:

➢ Obtention des valeurs des atténuations pour chaque projection,

➢ Calculs des valeurs d'un profil,

➢ Représentation matricielle de chaque plan de coupe,

➢ Traduction de chaque représentation par une carte particulière,

➢ Etablissement d'un système cartographique spatialisé.

On arrive aujourd'hui à des volumes de chaque zone élémentaire de l'ordre du mm$^3$.

Un tel exemple de scanner est décrit dans le document US5241471. **Selon ce document, on effectue 256 balayages radiologiques du corps en faisant tourner la source et le détecteur 256 fois autour du corps perpendiculairement au plan de coupe dont on veut réaliser l'image. Le détecteur comprenant 256 cellules, on mesure pour chaque balayage, une variation d'intensité des rayons X, entre la source et le détecteur, le long de $p=256^2$ profils de balayage. A l'aide d'un ordinateur dûment programmé, on calcule les $p=256^2$ valeurs associées aux $p=256^2$ points du plan de coupe. Ce calcul est effectué normalement par une technique de reconstruction algébrique (ART). Toutefois, l'amélioration prônée ici consiste à ne traiter d'abord qu'un sous-ensemble des $p=256^2$ profils de balayage, par exemple le quart, soit 64x256 profils de balayage. Ces profils résultent soit d'une moyenne par quatre des p profils de balayage réellement effectués, soit de la sélection d'un sur quatre de ces p profils. La technique de reconstruction algébrique est ensuite appliquée aux valeurs calculées à partir du sous-groupe de profils de balayage, pour aboutir à une image représentative des $256^2$ points du plan de coupe, soit la plus haute définition possible de l'image en fonction du détecteur utilisé.**

C'est cependant loin de l'échelle microscopique puisque le nombre de cellules vivantes est de l'ordre du milliard dans un mm$^3$.

La détection précoce du cancer suppose un gain considérable de la définition. Mais le temps d'utilisation du système pour un patient déterminé ne saurait dépasser un seuil économique évident. Mais surtout l'accroissement du nombre de profils augmente la dose d'irradiation.

On admet en effet qu'un nodule cancéreux accélère son développement lorsqu'il provoque une vascularisation associée, ce phénomène apparaît lorsqu'une taille critique est atteinte soit par exemple 50 microns. Dans les procédés classiques la dose d'irradiation et le temps de calcul sont multipliés par 8000, pour atteindre ce niveau de finesse.

C'est la raison pour laquelle l'auteur s'est d'abord efforcé d'obtenir un gain de la définition et de trouver une solution qui tienne compte de la capacité croissante des puissances de calculs et de la progression beaucoup plus lente de l'appareillage à RAYONS X.

A cet effet, l'invention a pour objet un procédé selon la revendication 1.

L'invention s'étend à un dispositif de mise en oeuvre d'un procédé selon la revendication 3.

Dans un mode particulier de réalisation de l'invention, un faisceau de rayon X, de lumière infrarouge ou d'ultrasons est déplacé pas à pas de manière linéaire, grâce à un guidage par des actuateurs piézoélectriques, de sorte à obtenir des coefficients relatifs à chaque profil correspondant à un pas donné pour réaliser un ensemble d'images, obtenues grâce à deux séries de profils, telles que dans chaque série les profils soient parallèles entre eux, et que les deux séries soient sécantes.

De préférence, le faisceau pivote autour d'un axe se déplaçant lui-même linéairement grâce à des actuateurs piézoélectriques. Des barrettes de détection multiples sont prévues pour réaliser simultanément plusieurs images parallèles susceptibles d'un traitement individuel ou en trois dimensions. Sont également prévues des plaques ou autres supports de faisceaux, de rayons X, ou de lasers, notamment à infrarouge, ou d'ultrasons, ou des champs magnétiques sur lesquels les actuateurs piézo électriques permettent d'orienter les faisceaux ou de les déplacer, en vue d'obtenir grâce à l'ensemble des détecteurs des informations concernant l'atténuation de chaque profil engendré par un pas du faisceau. Deux groupes de faisceaux peuvent fonctionner, l'un pour l'exploration, l'autre pour le traitement. Le dispositif peut comprendre également des plaques d'obturation perforées, lesquelles peuvent être occultées par deux réseaux croisés de fils ou lamelles déplaçables pour dégager une obturation ou plusieurs par déplacement des plaques ou lamelles.

Dans un autre mode particulier de réalisation de l'invention, un miroir est déplacé pas à pas de manière linéaire grâce à un guidage et déplacé en rotation autour d'un pivot, par des actuateurs piézoélectriques, pour réfléchir un faisceau laser à infrarouges de sorte à obtenir des coefficients relatifs à chaque profil correspondant à un pas donné du déplacement linéaire du miroir et réaliser un ensemble d'images obtenues grâce à deux séries de profils, les profils dans chaque série étant parallèles entre eux et les deux séries étant sécantes.

Dans le cas où l'on utilise un faisceau laser à infrarouge, on prévoit deux plaques transparentes, revêtues de couches anti-reflets pour éviter une réflexion du faisceau infrarouge et parallèles pour limiter une réfraction de ce faisceau, l'objet à examiner étant disposé entre les deux plaques et en appui contre l'une d'elle servant de plaque de support.

Ainsi la combinaison d'un appareillage à balayage fin, associé à des caches de très faible dimension, positionnables et commutables de manière ultra précise, et, d'un traitement de l'information permettant une évaluation acceptable des valeurs du coefficient d'atténuation de chaque micro zone élémentaire, permet de diviser par un facteur considérable le nombre de jets de faisceaux donc le nombre de profils à enregistrer, tout en portant la définition de un mm à 10 ou 20 microns , sans atteindre un niveau d'irradiation prohibitif.

En effet pour une matrice image 1000x1000 la résolution par la méthode standard nécessite un million de profils. Par la méthode exposée ci après il est possible dans un premier temps d'établir une image correspondant à une définition standard correspondant à une matrice 20x20 puis d'en déduire une deuxième image simplement extrapolée pour aboutir à une image 1000x1000, de balayer à nouveau le long de deux axes l'organisme étudié grâce à seulement 2000 profils pour obtenir une estimation convenable du coefficient d'atténuation de chacun des un million de points.

Selon ce procédé il suffit d'effectuer 400 profils dans une première étape puis 2000, soit au total 2400 profils, ce qui entraîne 99.76%, de réduction du nombre de profils nécessaire.

Le taux d'irradiation est dans ce cas divisé par 417, cette amélioration est encore plus significative pour des aires d'explorations importantes.

Pour un examen d'une zone de 20x20 cm, avec une définition de 1 mm, telle que pratiquée actuellement, on obtient les coefficients d'atténuation relatifs à 200x200 point soit 40 000 points, ce qui nécessite l'établissement de 40 000 profils. Si l'on souhaite obtenir une définition de 10 microns, il faut disposer des valeurs des coefficients d'atténuation d'un nombre de points 10 000 fois plus nombreux, soit 400 millions de points, et infliger au patient un taux d'irradiation létal, mais on est ainsi conduit à des délais et des coûts virtuels prohibitifs quelle que soit d'ailleurs le procédé utilisé, échographique ou par résonance magnétique.

Dans le procédé ici exposé, il est prévu de réaliser 40 000 profils pour obtenir l'image à faible définition, plus 40 000 profils supplémentaires pour le balayage croisé à haute définition soit au total 80 000 profils au lieu de 400 millions, soit une division par 5000 le nombre de profils et de l'irradiation associée.

Mais il reste à résoudre le problème du traitement du signal et des valeurs qui lui sont associées.

L'auteur a donc été conduit à mettre au point des méthodes de traitement du signal et de l'information, en vue de résoudre le problème particulier concernant l'évaluation des termes d'une matrice rectangulaire, représentatives des profils de découpage d'une aire déterminée explorée par un scanner lorsque l'on dispose d'estimations approchées relatives à chaque terme, mais d'informations précises concernant la somme de chaque ligne ou colonne de la matrice.

Plusieurs méthodes ont été mises au point et explorées pour résoudre le problème rencontré dans différents contextes de l'imagerie. Les recherches ont conduit à une méthode originale, laquelle permet une simplification considérable du processus de calcul, en vue d'obtenir l'image recherchée, représentée par une matrice rectangle.

L'ensemble des coefficients d'atténuation des points d'une aire quelconque située dans un plan d'examen, ou sur une surface, peut en effet être représentée par une telle matrice.

Elle permet dans le cas de l'imagerie médicale de limiter le nombre de profils à réaliser.

Cette méthode qui va ici être exposée permet en effet de produire des matrices de plus grande dimension par une répartition ou une extrapolation des valeurs de chaque terme ou zone d'une matrice initiale de faible définition pour obtenir des valeurs estimées dans des micro zones, résultant du découpage de chaque terme ou zone, de la matrice initiale.

On obtient ainsi une matrice amplifiée.

Cette matrice amplifiée est alors ajustée par un procédé de calcul permettant d'évaluer de manière fiable chaque terme si l'on peut établir les valeurs des éléments de bordure, un élément de bordure est la somme des termes d'une ligne ou d'une colonne.

Dans ce cas, on peut passer par exemple d'une matrice 5x4 contenant 20 termes en une matrice 25x20, contenant 500 termes, puis calculer chacun des termes par le procédé exposé.

Il y a bien alors, amplification et ajustement de la matrice initiale représentative des signaux obtenus pour la réalisation des différents profils.

La description du procédé d'ajustement est exposée ci-dessous. Ce procédé joue un rôle particulier dans le calcul effectué après, le cas échéant, obtention d'une matrice de grande dimension à partir d'une matrice initiale plus petite.

La description qui va suivre expose donc la méthode de calcul d'ajustement proprement dite selon l'invention Cette méthode joue un rôle important dans le traitement des signaux résultant de la mesure, par les détecteurs radiographiques, de l'intensité ou de la valeur résiduelle du faisceau élémentaire produit par le dispositif à rayons X, après le parcours dans l'organisme à étudier.

Si l'on veut traiter une matrice de dimensions n lignes et m colonnes

➢ si on appelle $B_{ij}$ la valeur estimée à la ligne $i$ et à la colonne j,

➢ si on appelle $C_{ij}$ la valeur la plus probable du terme correspondant de la matrice,

➢ si on appelle $\rho_j$ la somme des termes de la colonne j,

➢ si on appelle $c_j$ la somme des termes de la ligne $i$.

L'estimation de $B_{ij}$ résulte soit du processus d'amplification matricielle, soit de toute autre méthode permettant une telle estimation, notamment à partir des techniques de l'ajustement linéaire ou polynomial.

Dans le cas présent, on recherchera la solution des valeurs de $C_{ij}$ en tenant compte des contraintes de lignes et de colonnes, c'est à dire le minimum de la fonction :

$$\sum (Cij - Bij)^2$$

Pour toutes les valeurs de i et de j
sous les contraintes :

$$\sum_{i=1}^{n} Cij = \rho_j$$

pour toutes les valeurs de j

$$\sum_{j=1}^{m} Cij = c_i$$

pour toutes les valeurs de i

La recherche d'un minimum de la fonction sous contraintes sera faite en utilisant la méthode des multiplicateurs de Lagrange, le Lagrangien s'écrira :

$$L = - \sum_{ij} (Cij - Bij)^2 + \sum_{j=1}^{m} \lambda_j (\sum_{i=1}^{n} (Cij - \rho_j)) + \sum_{i=1}^{n} \mu_i (\sum_{j=1}^{m}$$

$$(Cij - c_i))$$

Cette fonction est composée de deux parties, une première n'a pas un caractère gauche et la deuxième est un ensemble de relations linéaires.

Le Lagrangien est donc dérivable pour les variables Cij et $\lambda_j$ et $\mu_i$, multiplicateurs de Lagrange associés aux contraintes de lignes et colonnes (nous disposons en effet de deux groupes de contraintes, les contraintes de ligne et les contraintes de colonne).

Dans ces conditions nous sommes en mesure d'obtenir un ensemble de relations linéaires concernant les Cij par dérivation du Lagrangien et un ensemble de valeur de relations relatives aux valeurs de contraintes, ce qui s'écrit :

En précisant que le dL/dCij désigne une dérivée partielle, de la fonction L pour la variable Cij.

$$dL/dCij = -2(Cij - Bij) + \lambda_j + \mu_i = 0 \qquad \underline{1}$$

et les contraintes

$$\left. \begin{array}{l} \sum_{i=1}^{n} Cij = \rho_j \ pour \ tout \ j \\ \\ \sum_{j=1}^{m} Cij = c_i \ pour \ tout \ i \end{array} \right\} \qquad \underline{2}$$

$$\underline{1} \Leftrightarrow Cij = Bij + (\lambda_j + \mu_i)/2$$

L'ensemble des n*m relations correspondant aux dérivées partielles plus les n+m relations de contraintes est linéaire et n'admet qu'une solution correspondant aux nm+n+m variables.

Si par exemple on veut traiter une matrice où n, le nombre de lignes, est égal à 25 et m, le nombre de colonnes, est égal à 30, la solution par l'algèbre linéaire consiste à traiter :

750 variables Cij

25 variables correspondant aux multiplicateurs de lignes, les $\mu_i$

30 variables correspondant aux multiplicateurs de colonnes, les $\lambda_j$

Un premier objectif est déjà atteint puisque seulement 55 profils doivent être établis au lieu de 750.

Nous disposons au total de 750 relations correspondant aux dérivés partielles et de 55 relations correspondant aux contraintes, pour 805 variables. La résolution de ce problème en faisant appel au calcul matriciel est la solution la plus évidente mais elle implique des calculs très lourds, légèrement plus lourds que ceux impliqués par les méthodes classiques. L'auteur a d'abord tablé sur l'amélioration rapide des processus de calculs, mais au delà de ce qui était son objectif essentiel, la limitation de taux d'irradiation en cours d'examen, il a poursuivi sa recherche pour tenter d'améliorer le temps de calcul.

De la combinaison des relations $\underline{1}$ et $\underline{2}$ on déduit :

$$\sum_{i=1}^{n} Bij + \frac{n}{2} * \lambda_j + (\sum_{i=1}^{n}(\mu_i)/2) = \rho_j$$

$$\sum_{i=1}^{m} Bij + \frac{m}{2} * \mu_i + (\sum_{j=1}^{m}(\lambda_j)/2) = c_i$$

On peut déduire de ces relations :

$$\lambda_j = \left(\frac{1}{n}\right) * (2 * (\rho_j - \sum_{i=1}^{n} Bij) - \sum_{i=1}^{n}\mu_i)$$

$$\mu_i = \left(\frac{1}{m}\right) * (2 * (c_i - \sum_{j=1}^{m} Bij) - \sum_{j=1}^{m}\lambda_j)$$

Dans ces conditions en reportant par exemple la valeur de λj dans μi, on obtient:

$$\text{Pour tout j } \lambda_j = \left(\frac{2}{n}\right) * ((\rho_j - \sum_{i=1}^{n} Bij) - \frac{1}{2} \sum_{i=1}^{n}\mu_i)$$

$$\text{Pour tout i } \mu_i = \left(\frac{1}{n}\right)(\sum_{i=1}^{n}\mu_i) + \left(\frac{2}{m}\right)(c_i - \sum_{j=1}^{m} Bij - \left(\frac{1}{n}\right) *$$

$$\sum_{j=1}^{m} \rho_j + \sum_{i}^{n} \sum_{j}^{m} Bij * \left(\frac{1}{n}\right))$$

Si l'on précise que $\mu^- = (1/n) \sum (i=1 \text{à } n) \mu_i$ soit la moyenne des multiplicateurs liée à la contrainte des lignes, on est conduit aux deux relations suivantes :

$$\text{Pour tout } j \quad \lambda_j = \left(\frac{2}{n}\right) * (\rho_j - \sum_{i=1}^{n} Bij) - \mu^-$$

$$\text{Pour tout } i \quad \mu_i = \mu^- + \left(\frac{2}{m}\right) * (c_i - \sum_{j=1}^{m} Bij - \left(\frac{1}{n}\right) * (\sum_{j=1}^{m} \rho_j - \sum_{i}^{n} \sum_{j}^{m} Bij))$$

En effet : $\frac{1}{m} \sum_{1}^{m} * \sum_{1}^{n} \mu_i$ est égal à $n*\mu^-$

Dans ces conditions en reportant dans la relation :

Cij = Bij + * $(\lambda_j + \mu_i)/2$ on est conduit à la relation algébrique

Cette formule d'ajustement permet de déduire la matrice des Cij de la matrice des Bij, par un calcul terme à terme

$$Cij = Bij + \left(\frac{1}{n}\right) * (\rho_j - \sum_{1}^{n} Bij) + \left(\frac{1}{m}\right) * (c_i - \sum_{j=1}^{m} Bij) - \left(\frac{1}{nm}\right) * (\sum_{j=1}^{m} \rho_j - \sum_{i}^{n} \sum_{j}^{m} Bij)$$

L'auteur a ainsi réussi à aboutir de manière tout à fait surprenante à un calcul de nature algébrique ne nécessitant pas le recours au calcul matriciel.

La méthode algébrique autorise le traitement partiel de la matrice de référence qui dans beaucoup de cas peut suffire.

La validation numérique de cette formule de traitement de signaux et d'établissement des valeurs de définition de l'image recherchée sur le plan médical, apparaît ci après.

*Exemple d'application de la méthode, sur un modèle réduit*

Soit donc une matrice de n lignes et m colonnes dans laquelle n=3, m=4

### TABLEAU 1 MATRICE INITIALE

|   | 1 | 2 | 3 | 4 | $\Sigma$ lignes | C |
|---|---|---|---|---|---|---|
| 1 | 22 | 24 | 18 | 16 | 80 | 78 |
| 2 | 24 | 22 | 18 | 20 | 84 | 85 |
| 3 | 26 | 20 | 22 | 24 | 92 | 93 |
| $\Sigma$ colonnes | 72 | 66 | 58 | 60 | 256 | |
| P | 70 | 67 | 59 | 60 | | 256 |

Dans cette matrice, les valeurs estimées sont inscrites sur les trois lignes et les quatre colonnes, les contraintes de lignes sont inscrites dans la colonne C.

Les contraintes de colonnes sont inscrites dans la dernière ligne P.

L'application de la formule ci dessus se simplifie puisque le total des contraintes de colonnes (ou de lignes) est égal à la somme des termes et conduit à:

**TABLEAU D' EQUILIBRE APRES CALCULS**

|  | 1 | 2 | 3 | 4 | ∑2 | C | Δ |
|---|---|---|---|---|---|---|---|
| 1 | 20.83333 | 23.83333 | 17.8333 | 15.5 | 78 | 78 | 0 |
| 2 | 23.5833 | 22.58333 | 18.5833 | 20.25 | 85 | 85 | 0 |
| 3 | 25.5833 | 20.5333 | 22.583 | 24.25 | 93 | 93 | 0 |
| ∑2 | 70 | 67 | 59 | 60 | 256 |  |  |
| P | 70 | 67 | 59 | 60 |  |  |  |
| Δ | 0 | 0 | 0 | 0 |  |  |  |

On peut vérifier, en tenant compte de calculs réalisés par une simple calculatrice que la valeur des termes sommés verticalement ou horizontalement satisfont les contraintes, mais aussi qu'il conduisent bien aux résultats recherchés.

Equilibrage en utilisant la méthode de l'algèbre linéaire

Cette méthode exprime directement les relations linéaires entre les variables $C_{ij}$ et $B_{ij}$, et les variables $\lambda_j$ et $\mu_i$.
La méthode classique de résolution d'un système linéaire implique l'inversion de la matrice des coefficients des relations entre les variables et la multiplication par cette matrice inverse, du vecteur exprimant les seconds membres des relations.

a) Il existe 12 relations entre les variables résultant de l'expression des dérivées partielles du LAGRANGIEN de la forme :

$$C_{ij} - \lambda_j / 2 - \mu_i / 2 = B_{ij}$$

b) Il existe 4 relations de contraintes relatives aux colonnes et 3 relations de contraintes relatives aux lignes, de la forme :

$$\sum_{i=1}^{m} C_{ij} = \rho_j$$

$$\sum_{j=1}^{n} C_{ij} = c_i$$

La méthode classique nécessite alors l'inversion d'une matrice d'une taille égale à n * m + n + m soit dans notre cas 19x19, dont le temps de calcul est évidemment beaucoup plus élevé.

L'analyse en deux étapes, correspondant à 6 séquences

A) L'amplification matricielle
Elle se déroule selon la séquence suivante :

1. Création d'une matrice initiale, traduisant une double série de profils croisés correspondant aux coefficients d'atténuation élémentaire d'une exploration tomodensitométrique; nous reprenons par exemple le tableau N° 1 « Matrice initiale » (page 11).

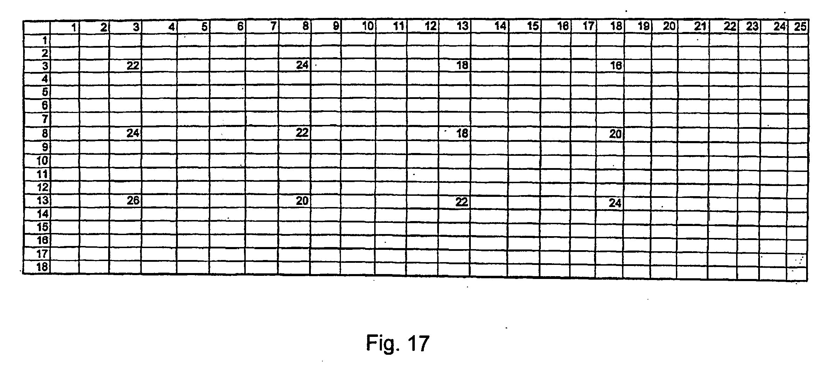

2. Mise en place d'un tableau de structure voir en annexe le tableau N° 2 intitulé « AVANT AMPLIFICATION », figure 17.

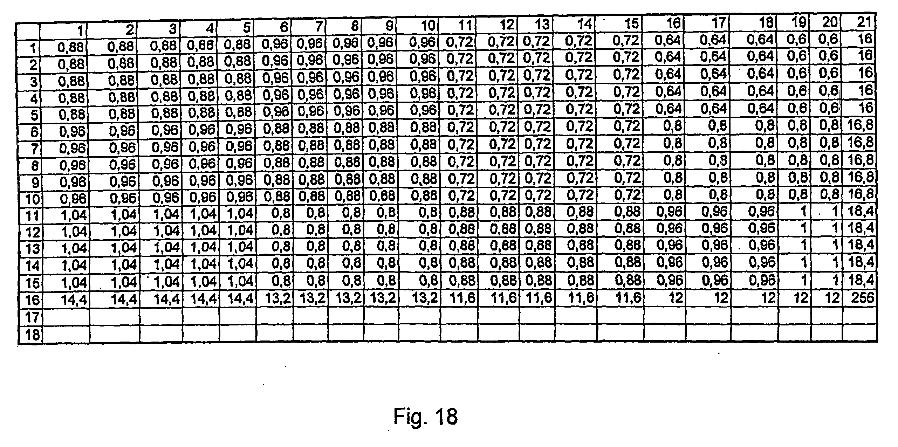

3. Inscription dans les cases à proximité de chacune des valeurs divisée par 25 ce qui conduit au tableau N° 3 « après AMPLIFICATION », figure 18.

B) L'ajustement se déroule ainsi :

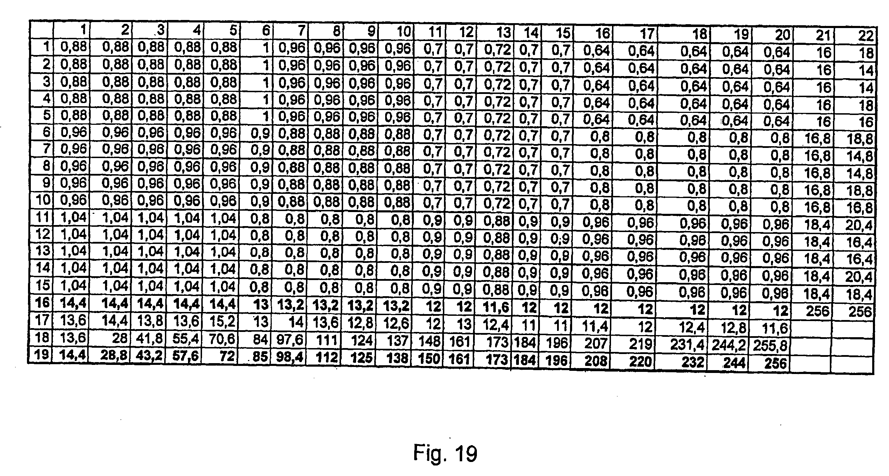

4. inscription des contraintes de lignes et de colonnes ce qui conduit au Tableau N° 4 « AMPLI + CONTRAINTES », figure 19, dans lequel les sommes des lignes et des colonnes, et les contraintes correspondantes sont indiquées. Chaque contrainte résulte de la mesure physique de la valeur totale d'atténuation d'un profil obtenu par lecture de la valeur atteinte sur le détecteur d'extrémité du profil, selon un balayage particulier.

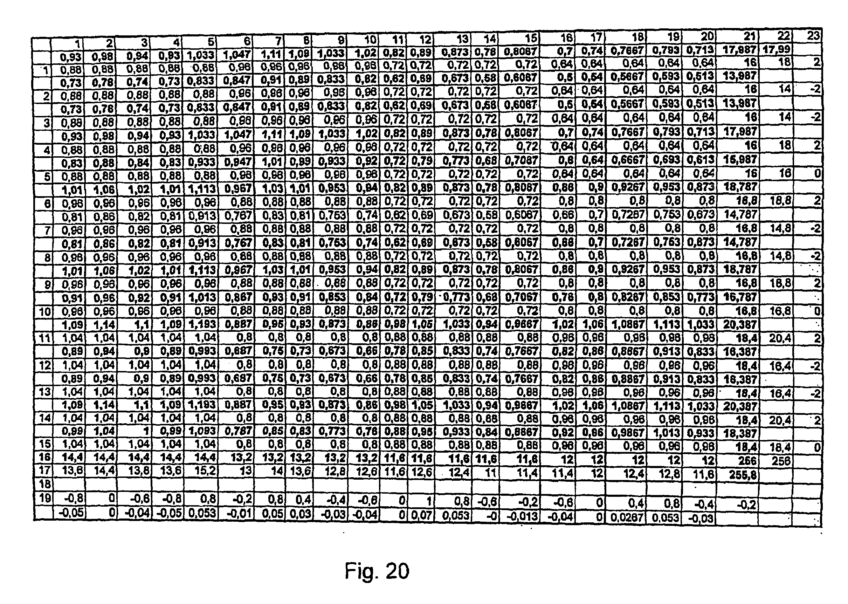

5. Calculs des valeurs par la formule indiquée auparavant, ce qui conduit au tableau N° 5 « AMPLI + CONTRAINTES + CALCULS », figure 20.

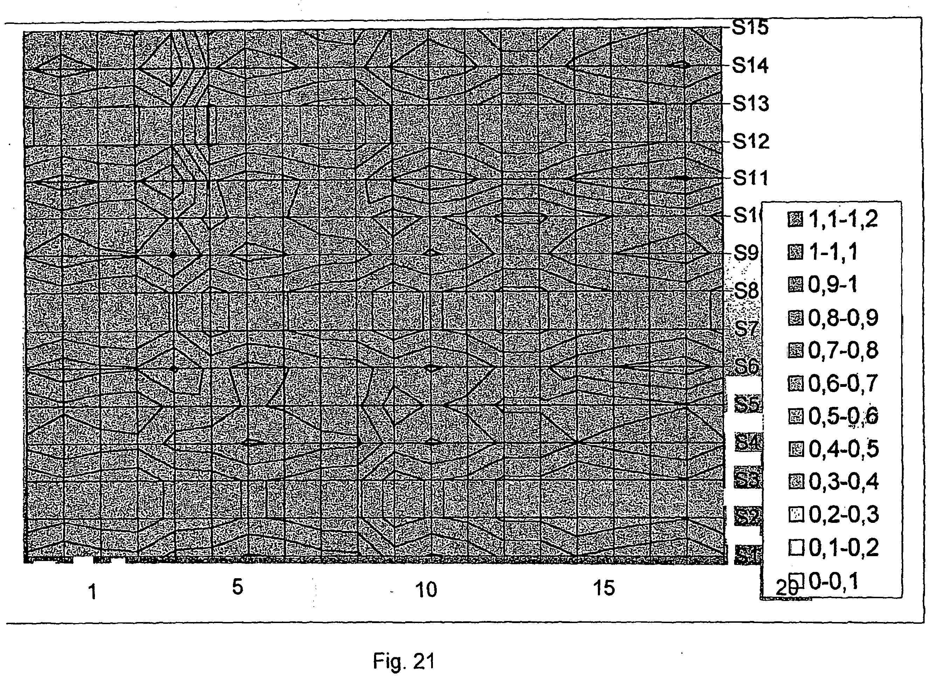

6. On peut alors traduire le tableau N°5, en ce qui concernent les valeurs des Cij, par une image colorée et graduée, constituant le tableau N°6-1, figure 21.

On a ainsi abouti au résultat recherché par le praticien d'une image médicalement utilisable dont la définition est meilleure avec un nombre limité d'opérations, et donc une quantité réduite d'irradiation telle que nécessaire par les procédés existants.

En réalité toute aire dans un plan, ou sur une surface, conduit à la création d'une matrice rectangle, cette matrice résulte simplement de l'établissement de deux séries de profils parallèles entre eux dans chaque série, que chaque série soit perpendiculaire ou non à l'autre. Elle peut aussi résulter des informations obtenues en coordonnées polaires, avec des séries profils passant par un pole donné, se recoupant le cas échéant avec des profils obtenus à partir d'un autre pole.

Rappelons qu'il est toujours possible de ne traiter qu'une partie de l'aire de référence sans aucune complication.

Il est, en effet, toujours possible de limiter le calcul à une ou plusieurs zones de l'image (Etant donné la nature même du procédé de calcul).

Les étapes du processus sont alors les suivantes :

a) on met en oeuvre un examen avec un balayage à définition classique par exemple 1 mm

b) On obtient une image appropriée

c) On définit une aire d'exploration fine

d) On met en oeuvre un balayage à haute définition par un dispositif approprié, qui suppose des modes de déplacement spécifiques

e) On procède au traitement de l'information de l'aire choisie ce qui suppose :

1) L'estimation des valeurs d'atténuation des micro zones résultant de la division des zones élémentaires, de manière raisonnée, et ce à partir d'une image de référence obtenue par un examen avec un balayage à définition classique,

2) Le calcul des valeurs de termes de la matrice correspondant à l'aire déterminée, par la méthode d'ajustement exposée précédemment, sur la base du balayage à haute définition,

3) Le cas échéant on peut décider la réitération du processus, fondée sur un changement total ou partiel de l'échelle du découpage, ou de la zone explorée, ou le cas échéant sur la réitération du balayage sous des angles différents de la même zone pour obtenir plusieurs valeurs de l'atténuation de chaque micro zone, dont la moyenne procure une évaluation améliorée et peut être associée à un écart type.

f) La création d'une base de données des valeurs d'atténuation de chaque micro zone dans les différentes phases de l'examen.

g) L'édition, partielle ou complète des résultats, et la visualisation qui en découle de manière plane ou en trois dimensions, en noir gris et blanc ou en couleurs.

On peut, si besoin est, réaliser un traitement focalisé, utilisant les mêmes techniques de balayage fin et le cas échéant l'appareillage d'exploration, si la puissance nécessaire doit devenir plus élevée, ce qui est le cas. Il est alors possible d'utiliser un faisceau laser X, (ou tout faisceau de faible section) lequel sera utilisé à plusieurs reprises à un niveau approprié d'amplification de sorte à associer exploration et traitement en diminuant le risque d'irradiation des organes non concernés.

En vue d'obtenir l'information nécessaire au traitement qui suivra on peut procéder de deux manières :

1 - Avec deux dispositifs associés

a) On peut en effet réaliser une analyse tomodensitométrique avec un scanner existant, comportant une couronne, autorisant un nombre suffisant de balayages, pour permettre une définition de l'image de un mm. On conservera l'information dans la mémoire du système informatique, on évaluera ensuite la valeur des coefficients d'atténuation de chaque micro zone résultant d'une division raisonnée dans chaque zone, et en limitant de manière précise l'exploration supplémentaire.

b) Puis on effectuera un balayage à haute définition, dans un deuxième appareil spécialisé de sorte à obtenir, dans une aire déterminée la valeur des coefficients d'atténuation, le long de rayons du faisceau beaucoup plus rapprochés. La configuration de ce deuxième appareil peut être identique à celle du premier, mais les balayages peuvent être limités à des zones angulaires plus petites, l'angle est alors fonction du nombre de pas à réaliser, pour obtenir un ensemble de mesures croisées suffisantes. Le deuxième appareil peut être plus simplement rectangulaire pour matérialiser physiquement une matrice. Dans ce cas deux faisceaux se déplacent perpendiculairement pour établir directement une matrice rectangle qui sera soumise au traitement mathématique de redressement dont la nature a été exposée.

c) Le problème est aussi un problème de repérage pour que les zones puissent être traitées par superposition exacte.

Deux solutions peuvent alors être mises en oeuvre

➢ Une solution par analyse des images obtenues par les deux dispositifs, l'un et l'autre rotatif en utilisant un logiciel permettant une comparaison précise des coefficients d'atténuations dans chaque image de sorte à permettre une superposition des images obtenues, à la même échelle.

➢ Une autre solution en positionnant par exemple sur la peau des repères physiques, tels que grain de plomb ou d'autres matériaux ayant des propriétés faciles à détecter. Ces grains sont l'équivalent de bornes topographiques.

2 - Avec un dispositif intégré
Ce dispositif permet un fonctionnement à double balayage par exemple :

a) un balayage à déplacement de faisceau, avec un pas de l'ordre de 1 mm, pour obtenir une image telle que celle produite par un scanner de type actuel

b) séquentiellement (ou simultanément) un balayage à déplacement très limité du faisceau (avec un pas de un dixième à un centième de mm) pour sur la même image produire les deux catégories d'information sur la base de la même topographie

Un dispositif décrit ci après autorisera l'utilisation de l'un et ou l'autre des procédés de balayages au prix d'une certaine complication dans la mise en oeuvre des deux balayages, surtout lorsqu'il sont effectués simultanément.

On pourra, dans ce cas aussi, compléter le calage des images par repérage physique, en plaçant sur la peau, ou même dans l'organisme des matériaux à très forte absorption.

En effet on peut injecter des molécules susceptibles de se concentrer, dans les cancers ou dans les zones de vascularisation associées.

Dans le cas du dispositif intégré il suffirait de réaliser un système physique rectangulaire stable, supportant les plaques d'obturation et de détection, associé à deux faisceaux mobiles, se déplaçant perpendiculairement, en s'inclinant, l'un et l'autre indépendamment pour couvrir chacun une zone large de par exemple 90 ° d'inclinaison.

Ou en se déplaçant tout en gardant une position angulaire donnée, la combinaison des déplacements pouvant être ajustée selon des protocoles appropriés au cas à traiter. L'objectif final est de disposer de deux ensembles de données :

a) Une matrice de référence à balayage large, permettant d'identifier chaque zone

b) Une matrice, résultant des informations produites par balayage fin, susceptible d'être traitée, par les méthodes mathématiques exposées au début de la description du procédé, pour évaluer la valeur des coefficients d'atténuation dans chaque micro zone.

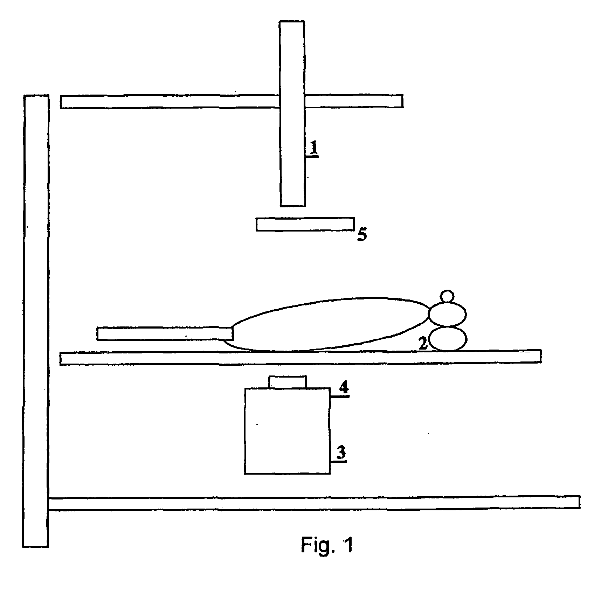

La planche N° 1 intitulée «Schéma d'ensemble» montre un exemple de réalisation. Dans cette planche, figure 1, l'accélérateur de particules ou faisceau 1 peut se déplacer. Le plateau de positionnement 2 est déplaçable en hauteur et translatable horizontalement 9. La plaque d'absorption 3 est composée de matériaux lourds pour éviter l'effet des rayonnements. La plaque support des barrettes de détection 4 supporte un ensemble de barrettes permettant la lecture des coefficients d'atténuation en l'absence ou en présence du corps ou de l'objet à examiner. L'obturateur 5 est composé d'un ensemble d'orifices à position et ouvertures ajustables.

Ce schéma représente une coupe de l'ensemble du système physique. La table 2 sur laquelle repose le patient est placée à l'intérieur d'un cadre. Le faisceau 1 vertical, traverse une plaque d'obturateurs 5 puis le patient, puis la table, pour atteindre la plaque 4 supportant les détecteurs, laquelle est reliée au dispositif informatique.

Nous avons choisi de représenter le cas nouveau, d'un cadre rectangulaire, associé à au moins deux faisceaux inclinables et déplaçables.

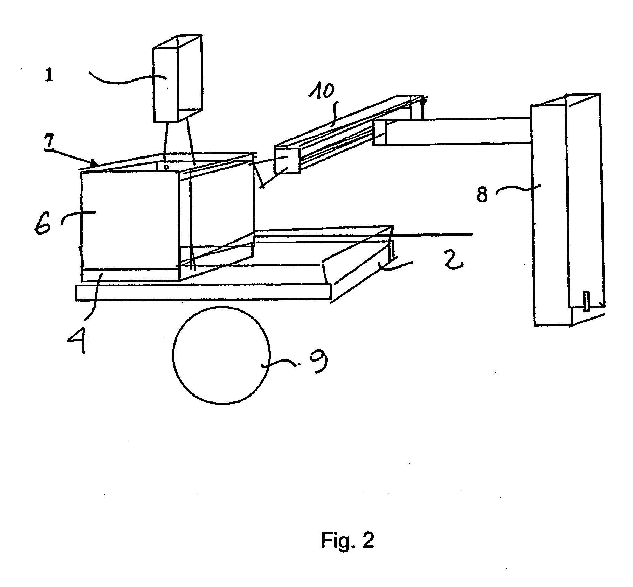

La Planche N° 2 intitulée «Faisceaux et obturateurs, vue perspective» est une projection de l'ensemble montrant les balayages verticaux et horizontaux vus en perspective, dans une variante, dans laquelle le cadre est posé sur la table et le patient installé sur un matelas; pour lui permettre de pénétrer dans le cadre. Dans cette planche, figure 2, 1 désigne un faisceau vertical, 10 un faisceau horizontal, 2 une table de positionnement, 8 une potence supportant le faisceau horizontal, 4 une plaque de détection horizontale, 6 une plaque de détection verticale et 7 une grille d'obturation horizontale.

Dans cette variante le faisceau ne traverse pas la table, cette disposition a pour objectif d'éliminer dans le traitement de l'information, le problème de ladite table.

Il est bien entendu possible d'utiliser des faisceaux à puissance normale pour limiter les risques, dus au rayonnement. Dans ce cas le traitement supposera que l'on dispose sur le même site des deux types de faisceaux , ou d'un laser X fonctionnant à plusieurs niveaux d'amplification.

Pour que l'organisme reçoive le faisceau, de manière précise, on place dans le trajet une plaque de plomb dans laquelle une fenêtre est ouverte; dont la dimension et la découpe est réalisée en fonction de chaque protocole d'analyse ou de traitement envisagé.

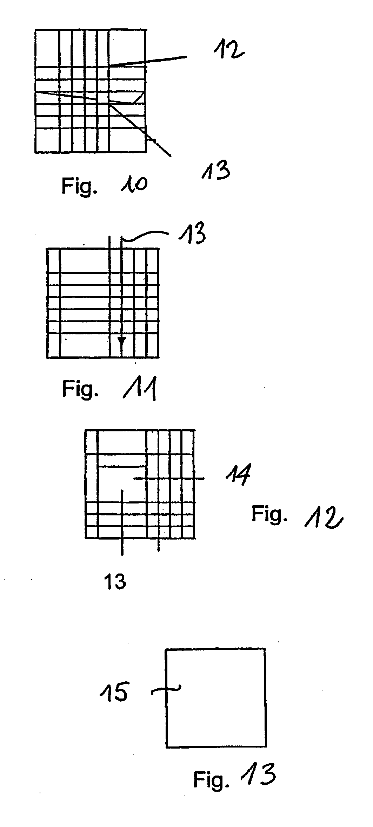

Dans le cas de notre système on installe au surplus une plaque contenant des orifices très petits de un à deux centièmes de millimètres; ces orifices peuvent être obturés préférablement par déplacement de fils très fin. On constitue ainsi une grille de deux ensembles superposés de fils, horizontaux 12 et verticaux 13, figure 10, se croisant à angle droit. Ces deux ensembles obturent simultanément les orifices. Pour une définition de un mm, les fils sont remplacés par des lamelles de largeur 0.8 à 1 mm, voir planche 5 «Système d'obturation», figures 10 à 13.

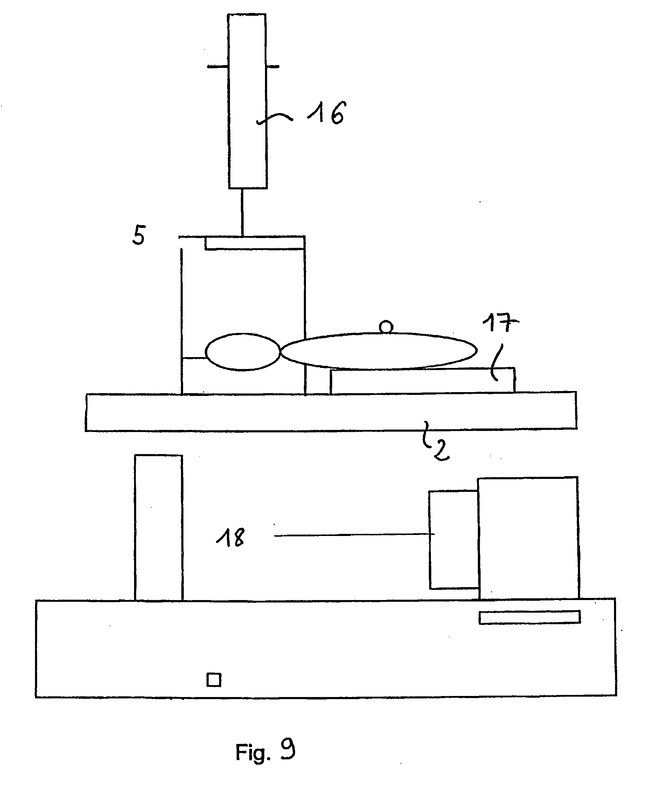

Le déplacement de deux fils, commandés en abscisse et en ordonnée dégage un seul orifice; les déplacements simultanés de deux sous ensembles dégagent une aire rectangulaire (voir la planche 5, figure 10). La figure 9 montre des fils 13 translatés horizontalement. La figure 10 montre une ouverture 14 après une translation verticale des fils 13.

Ces déplacements sont obtenus de manière précise par l'utilisation d'actuateurs piézo-électriques. Le dégagement d'une aire précise est effectué pendant un temps très court, de sorte à découvrir les micro zones à examiner ou traiter en une ou quelques milli secondes. Un boîtier 15 commande les déplacement des fils, figure 11.

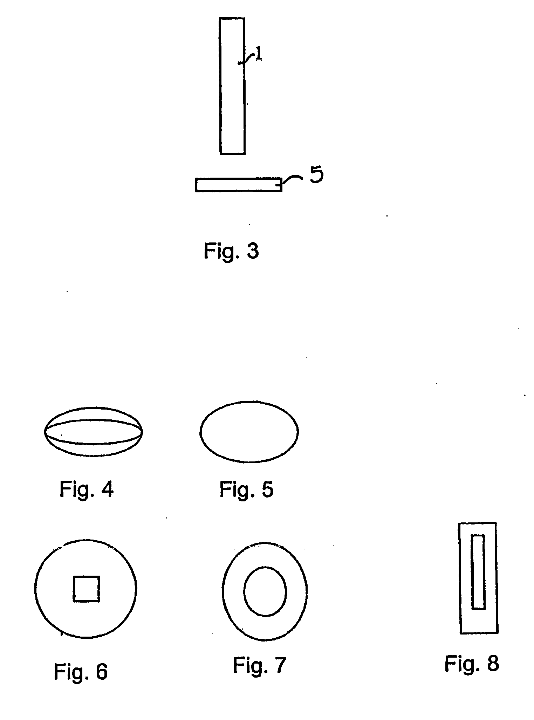

Voir aussi la planche N° 3 intitulée «Faisceau et obturation». Dans cette planche, figure 3, les obturateurs 5 du faisceau 1 sont composés d'une série de lucarnes fonctionnement simultanément ou indépendamment, groupées par blocs et déplaçables. Différentes formes d'obturateurs sont représentées par les figures 4 à 8. La figure 8 montre plus particulièrement une fente à section variable.

Pour éviter les difficultés inhérentes à de tels procédés, il sera préférable d'utiliser un laser à double fonction de positionnement et de visée, analogue à ceux utilisés pour des mesures de distance et de positionnement topographique.

Le schéma et la description sont donnés sur la planche N°4 intitulée «Laser de repérage». Un tel laser est utilisable lorsque l'on traite une fraction de l'organisme transparent comme l'oeil, ou si l'on veut examiner une surface comme la peau ou un organe interne pendant une intervention chirurgicale.

L'utilisation de plaques d'obturation et de détection et de véritables grilles à fonctionnement très rapides est destinée à permettre un cadrage de l'aire à examiner sans déplacement physique de l'objet ou du patient. Dans la planche 4, figure 9, le laser de repérage 16 occupe la position exacte du faisceau X. Le cadre portant le système d'obturation 5 est dans la trajectoire du rayon. La table support 2 supporte aussi le cadre d'obturation et celui de détection. Le matelas de repos 17 s'ajuste sur le niveau du cadre détection. L'armoire électrique 18 relaie toutes les informations au système informatique.

La Planche N° 6 intitulée «Analyse du balayage» montre la méthode de balayage utilisée.

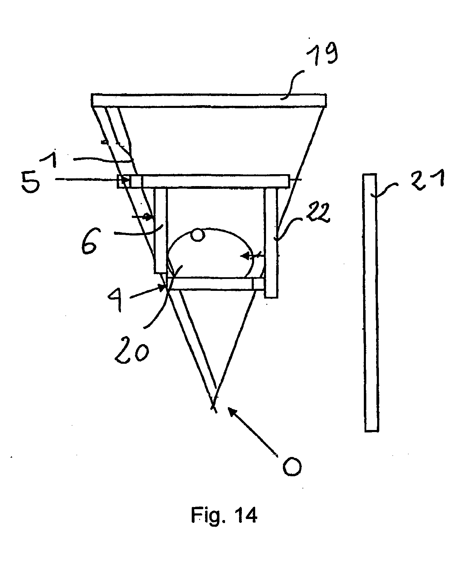

Figure 14, le faisceau 1 se déplace le long d'une plaque de balayage 19, il effectue une translation pas à pas. Dans le cas de la méthode classique, à chaque pas il s'incline progressivement de sorte à ce que les faisceaux successifs convergent vers un point central O. Chaque balayage permet au faisceau de traverser la plaque d'obturation 5, puis la zone à examiner 20, enfin d'atteindre le détecteur 4 dans une zone alignée dans l'axe défini par la position de l'articulation de faisceau avec la plaque et par l'angle de tir.

Le nombre de profils à obtenir doit être égal au nombre de points d'examens élémentaires, pour déterminer avec certitude le coefficient d'atténuation de chaque zone.

Une bonne approximation peut être obtenue, par une séquence de balayages fins tels que l'on obtienne un nombre suffisants d'estimations de chaque micro zone pour calculer une moyenne et un écart type convenables, pour chaque terme de la matrice amplifiée.

Dans le cas du balayage fin, l'inclinaison reste constante, pour l'un 1 et l'autre 10 des faisceaux, ou pour le même faisceau, si, figure 14, les plaques de support 19,21 du faisceau, de l'obturateur 5,22 et du détecteur 4,6 passent de la position horizontale 19,5,4 à la position verticale 21,22,6 en effectuant un déplacement pour procéder à deux séries d'analyse permettant aux deux balayages de se recouper dans toute la zone à examiner.

Le système fonctionne donc en quatre étapes pour le balayage fin

1. Définition de l'aire à examiner par exemple 4x6 mm,

2. Positionnement de cette aire en définissant les abscisses et les ordonnées des quatre sommets,

3. Allumage du faisceau approprié, accélérateur de particules pour le traitement, ou de rayons X pour l'analyse, ou Laser X fonctionnant à plusieurs niveaux, un niveau faible pour l'exploration, et plus élevé pour le traitement,

4. Désobturation rapide par déplacement selon deux axes de coordonnées des fils d'obturation.

L'information provenant des détecteurs est transmise en temps réel au système informatique, dans le cas de l'utilisation du même dispositif pour les deux phases de l'opération, qui supposent :

➢ un balayage avec un pas de un mm pour l'ensemble de l'examen

➢ puis un balayage avec par exemple un pas de vingt microns

Le premier balayage sera réalisé en déplaçant et inclinant le faisceau selon un protocole standard, les obturateurs s'ouvrant pour permettre l'examen de zones prédéfinies dans différentes conditions standard. Pour une aire de 10x10 cm, on réalisera 10'000 profils pour calculer de manière précise les valeurs de 10'000 points par inversion d'une matrice 10 000x10 000; ou dans des conditions beaucoup plus rapide si l'on se contente des valeurs moyennes associées à des écarts types, pour une série de mesures traitées par la méthode d'ajustement exposée précédemment. Cette procédure limite en particulier l'irradiation.

Pour le deuxième balayage, les deux faisceaux pourront préférablement garder leurs inclinaisons et se déplacer l'un et l'autre dans un champ limité pour permettre l'exploration d'une aire par exemple rectangulaire de 4x6 mm, on est conduit à générer (pour une définition de 20 microns) une matrice rectangle de 200x300, par plan de coupe soit 60 000 points, dont le traitement numérique restera dans des limites convenables, compte tenu de l'algorithme déjà exposé, tout en limitant l'irradiation.

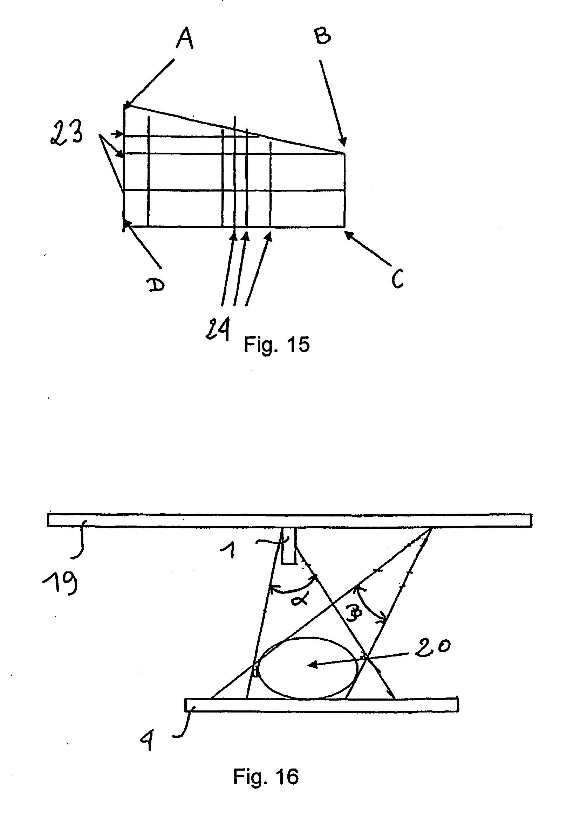

La planche 7 intitulée «Balayage spécial» présente un intérêt particulier, elle montre qu'il est possible d'utiliser une seule plaque de balayage 19, par exemple horizontale, et un seul plan de détection 4 ici horizontal. Figure 15, on a représenté un trapèze de sommets A, B, C et D. Les références 23 et 24 désignent respectivement des lignes horizontales et verticales.

Dans ces conditions, figure 16, l'objet 20 est balayé, dans le plan tomographique, par exemple sous deux angles $\alpha$ et $\beta$ tels que les balayages se recoupent en un nombre suffisant de points pour obtenir une évaluation pour chaque point éloigné du plus proche de 1 mm, et obtenir ainsi une évaluation précise.

Ce travail sera complété par au moins deux balayages par translation avec des pas de 20 microns environ ou par rotation, avec une fraction de degré par pas, pour obtenir les données nécessaires au traitement par la méthode déjà exposée. Les angles des deux groupes de balayage peuvent être ou non égaux à 90°. La complication résultant de l'utilisation d'axes de coordonnées non orthogonaux est négligeable sur le plan du calcul.

Nous rappelons que le dispositif peut comprendre un faisceau LASER A RAYONS X dont le rayon va traverser l'objet ou le patient à examiner. Après la traversée un détecteur permet de lire la puissance résiduelle du faisceau en vue de mesurer le coefficient d'atténuation pendant la traversée de l'objet ou du patient examiné.

Ce faisceau progresse point par point devant une batterie d'obturateurs, pouvant être constituée par des petits caches déplaçables en face d'orifices de très faible section.

Il est possible d'utilise un faisceau suffisamment large pour couvrir une batterie d'obturateurs, et affecter une barrette homologue de détecteurs.

Si l'on admet que certains déplacements naturels (par exemple la respiration) puissent déplacer des éléments d'organe à une vitesse de 1 cm par seconde; et si l'on veut obtenir une précision de l'image de 10 à 20 microns, le temps de fonctionnement de l'obturateur doit être de l'ordre de la milli seconde, et le dispositif doit créer une série de flashes successifs de très courte durée.

Si donc le faisceau doit être établi et interrompu très rapidement (dans le cas notamment de l'utilisation d'un laser lumineux ou à rayons X), ou si l'obturation doit être modifiée rapidement le fonctionnement de la commande (électrique ou mécanique) pendant une période très courte se heurte aux limites des systèmes électromagnétiques, sur le plan technique ou des systèmes électroniques, sur le plan des coûts.

Dans un brevet européen n° 99401358 déposé par Norbert BEYRARD, concernant «un contacteur à commande piézo électrique» l'auteur décrit un dispositif de commande d'un contacteur disjoncteur dont les déplacements sont effectués par un actuateur piézoélectrique.

Les essais les plus récents montrent :

➢ Que la coupure du courant peut s'effectuer en un temps de l'ordre de la milli seconde.

➢ Que la coupure de courant peut grâce à un circuit électronique approprié, s'effectuer lorsque le courant passe, par exemple en courant alternatif, au point ou l'intensité est nulle.

On peut sans extrapoler prévoir un dispositif de commande comportant

➢ un alternateur (ou un moduleur) délivrant un courant de fréquence égal par exemple à 200 ou 400 Hertz.

➢ un circuit électronique permettant que la mise en circuit s'effectue au moment d'un premier passage du courant à intensité nulle, et que la coupure s'effectue au passage suivant.

Dans ces conditions, le dispositif sera allumé, ou désobturé, pendant une demi période égale à 1/800$^{ème}$ de seconde, soit 1.25 milli seconde, ou de 1/400ème de seconde ou 2.5 milli secondes.

Dans le dispositif mis au point par Norbert BEYRARD et qui comprend un circuit électronique de commande, il est possible d'enclencher, puis de déclencher le circuit électrique pour que la période de fonctionnement soit ajustée de manière précise, tout en maintenant le fait que l'ouverture ou la fermeture du circuit s'effectue au moment où l'intensité du courant alternatif (sinusoïdal) passe par le point d'intensité nulle.

Il est ainsi possible de provoquer l'ouverture et la fermeture d'un circuit à un moment précis dans le temps.

Si donc le déplacement de l'objet ou de la zone à examiner s'effectue à 1 cm par seconde, il pourra être examiné au cours d'une période de 1 à 2 milli seconde, correspondant à un déplacement de l'ordre de 10 à 20 microns. Pour tenir compte des aléas le faisceau serait d'un diamètre de 100 microns (ce qui est normalement le cas pour certains laser X).

Il s'agit là d'un point essentiel qui autorise la recherche d'une définition de l'ordre de 10 à 20 microns au lieu de 1 mm actuellement, soit un gain de 50 à 100 de la définition par rapport à la technique actuelle.

Le gain économique qui en résulte peut s'exprimer simplement.

Un tel dispositif de coupure, tel que décrit dans le brevet précité, ne devrait coûter qu'une dizaine d'euros, pour une fabrication en série ayant éventuellement d'autres objets; la coupure électronique par exemple par un thyristor suppose un système de coupure dont le prix est environ cent fois plus élevé.

Si l'on dispose de systèmes ayant une telle précision, la quantité d'informations produite par le détecteur est multipliée dans des proportions considérables et dépasserait rapidement la limite logiciels et des calculateurs généralement associés aux scanners actuels.

Les calculateurs associés au nouveau dispositif devraient traiter un ensemble d'équations linéaires et de variables 125 000 fois plus important pour un gain de définition de 50.

Prenons un objet dont la section est de 20 cm sur 20 cm pour une zone de 1 mm. On est conduit à calculer 200 x 200 coefficient d'atténuation par section, soit 40.000 points. En dépit des astuces de programmation le temps et le coût des calculs sont encore aujourd'hui considérables.

Si l'on améliore la définition dans un rapport de 50 il faut multiplier dans une proportion considérable (de l'ordre de 125 000) la vitesse; et donc la puissance de calcul actuelle.

Si l'on considère que la Loi de Moore continuera à s'appliquer dans le futur, que la puissance de calcul double donc tous les 18 mois, il faudrait attendre 25 ans pour que les systèmes deviennent opérationnels.

C'est la raison pour laquelle il est nécessaire d'utiliser un autre procédé tel que décrit précédemment, en vue d'accélérer le processus.

Le processus de calcul est alors le suivant :

Au cours d'une première étape on s'efforce d'estimer les coefficients d'atténuation de zones réduites.

Admettons que l'on explore une zone limitée prise à l'intérieur d'une section explorée de manière classique, et que cette zone ait par exemple 4 mm sur 3mm. Dans une telle zone nous avons 12 zones, pour lesquels différents coefficients d'atténuation ont été calculés, de manière classique ou non.

La représentation de valeur de chaque zone est exprimée dans le tableau 1 «Matrice initiale» page 2.

Chaque zone est alors découpée en vingt cinq (5x5), c'est la raison pour laquelle dans le tableau de la figure 17 «AVANT AMPLIFICATION» la valeur du coefficient d'atténuation apparaît au centre de chaque zone.

Le coefficient relatif à la zone initiale N° 1 amplifiée est égal à 1/25, de 22 soit 0.88, on voit, sur le tableau de la figure 18 «Matrice après amplification» la matrice amplifiée, qui montre les coefficients d'atténuation de chaque micro zone après répartition.

Les valeurs des contraintes sont indiquées au tableau de la figure 19 «Après ampli + contraintes», et complètent les informations produites au tableau de la figure 18.

Nous avons testé la méthode d'ajustement décrite auparavant sur la base du tableau de la figure 19, et vérifié son fonctionnement en établissant le tableau de la figure 20 «ampli + contraintes + calculs», qui montre que la somme des coefficients d'atténuation estimée est bien égales à la valeur de la contrainte.

L'examen de ce tableau 5 permet de suivre le processus de calcul :

1. Les lignes numérotées indiquent la valeur estimée de chaque terme, elles sont les mêmes que celles homologues du tableau 4 (Les Bij)

2. La colonne 21 indique la somme des valeurs estimées du tableau

3. La colonne 22 les contraintes de lignes

4. La colonne 23 l'écart entre les valeurs de 22 et 21

De la même manière en bas du tableau apparaissent les différences entre contraintes de colonnes et somme des valeurs estimées pour chaque colonne.

Enfin, sur toutes les lignes en gras situées au dessus des valeurs des lignes numérotées, apparaissent les valeurs calculées grâce à une macro instruction qui lie les valeurs (Cij) résultant du calcul, aux valeurs indiquées sur les lignes numérotées (les Bij).

Par exemple :

Si l'on estime les coefficients indiqués en ligne 3, tirés de la matrice d'origine et ceux apparaissant dans la ligne au dessus, on vérifie sur la colonne 21 que la somme est bien égale à 13,987, soit une valeur très proche de celle de la contrainte égale à 14 qui apparaît dans la colonne 22.

Ce calcul effectué pour l'ensemble des coefficients du tableau montre que la méthode ainsi découverte permet le calcul dans des conditions d'extrême rapidité ; puisqu'une simple formule algébrique se substitue aux formules matricielles résultant normalement de ce processus de calcul.

Les calculateurs actuels peuvent traiter le problème correspondant et même aller bien au delà en appliquant des processus de calcul similaires à ceux exposés précédemment.

1. Lorsque l'on procède au calcul d'équilibrage avec un tableau obtenu par répartition des valeurs de chaque zone en par exemple 25 micro zones, on peut se trouver loin des valeurs de contraintes de ligne ou de colonnes de deux manières :

2. Si l'on ne dispose pas des valeurs de bordures, celles ci peuvent être évaluées par répartition des valeurs connues sur la ligne ou la colonne, grâce à l'étape de définition standard (1 mm).

3. On peut procéder alors à un calcul pas à pas avec des pas réduits à 20 microns et des temps de fonctionnement de l'ordre de la milli seconde. Cette méthode mettra en évidence les écarts entre estimation et observation. Le cas échéant, on effectuera plusieurs balayages fins sous des angles différents, on disposera ainsi de plusieurs évaluations des Cij, conduisant à une amélioration de la valeur recherchée.

4. Si les écarts entre estimation et observation sont inférieurs à un seuil fixé à l'avance, on admettra par exemple un écart relatif de 20 %, et on procédera sans risque au calcul par les méthodes exposées ci dessus.

5. Si les écarts par contre sont plus élevés et s'ils restent inférieurs à un deuxième seuil, par exemple +/- 40% d'écart relatif, on diminuera le taux d'amplification en passant par exemple d'une division de chaque zone en 25 micro zones à une division de chaque zone en 9 micro zones. Si en dépit de cette nouvelle répartition des écarts persistent à l'intérieur de l'aire considérée, on procédera à un traitement selon les modalités classiques pour calculer les coefficients d'atténuation avec certitude, ou de manière plus économique par la méthode utilisant une séquence de balayages.

6. Il est donc possible aujourd'hui d'envisager de traiter avec des calculateurs puissants mais disponibles, des mini zones de 1 cm x 1 cm, soit 250 000 micro zones de 20 microns de coté, au lieu de 1 mm (la résolution actuelle),

7. Cette technique de résolution conduit à une amélioration de la définition d'un facteur 50, et ce en tenant compte des limites physiques de la dimension de l'obturateur et temporelles du fonctionnement du faisceau ou l'interruption de l'obturateur.

8. L'exemple des tableaux 3, 4 et 5 proches de ce qui pourrait être envisagé conduirait à mesurer 35 valeurs de bordures du faisceau pour 300 valeurs estimées des coefficients d'atténuation.

9. Le gain sur le plan économique et de santé est encore plus spectaculaire avec des tableaux plus importants.

On est cependant encore loin de la situation idéale permettant d'atteindre le niveau d'une micro zone de 1 micron, c'est à dire la taille d'une cellule.

Chacune des micro zones de 20 microns contient en effet 8 000 cellules.

Dans l'immédiat, ces calculs pourraient atteindre la limite des calculateurs associés au scanner, pour des examens de zones importantes, par exemple 20 x 20 cm.

Il est à nouveau rappelé, qu'il est possible d'effectuer dans la même zone, plusieurs balayages, par exemple une dizaine, qui donneront pour chaque point, une dizaine de valeurs voisines dont on pourra ainsi calculer la valeur moyenne et l'écart type, ce qui donne dans la plupart des cas, une estimation tout à fait convenable et évite le recours au calcul matriciel qui entraîne des temps de calcul prohibitifs.

Pour être utilisée, l'information doit être éditée de manière appropriée. On peut procéder de manière habituelle en éditant des cartes faisant apparaître pour chaque coupe des zones plus ou moins grises en fonction du coefficient d'atténuation, et ce de deux manières :

➢ de manière standard (ou par la méthode des balayages multiples) pour l'ensemble de la coupe avec une résolution de 1 mm

➢ de manière complémentaire pour chaque mini zone traitée plus en détail avec différents tons de gris, ou différentes couleurs.

On prévoira d'agrandir éventuellement les mini zones pour faciliter la lecture.

On prévoira avantageusement de tracer des lignes d'iso atténuation (courbes rejoignant des micro zones de même niveau d'atténuation) pour voir apparaître les anomalies particulières.

Grâce à un traitement coloré on peut envisager superposer les lignes d'iso atténuation sur les fonds à gris variables.

La planche 6-1 en annexe montre un exemple saisissant puisque outre la coloration, des lignes de niveaux d'atténuation sont établies, ce qui affine encore le résultat de l'investigation.

Il faut aussi tenir compte du balayage axial, c'est à dire du déplacement du plan de coupe, qui ne nécessite plus aujourd'hui de véritable déplacement physique, puisqu'on peut disposer des barrettes comprenant un nombre suffisant de détecteurs sur une longueur de plusieurs centimètres.

Le problème est aussi un problème de traitement de l'information puisque le déplacement axial devrait être du même ordre de grandeur que le balayage dans un plan de coupe, ce qui conduit à des calculs qui même largement simplifiés par les méthodes exposées, ne doivent pas devenir prohibitifs.

Lorsque pour des raisons d'exploration fine on sera conduit à rapprocher les coupes on pourra réaliser une projection à trois dimensions des informations, particulièrement pour les micro zones, tout aussi bien que pour des zones correspondantes à la définition actuelle.

L'auteur est donc conduit à prévoir un système opérant ainsi :

Il faut ainsi :

a) Faire fonctionner un scanner avec une résolution de 1 mm pour obtenir des mesures relatives à des zones de même dimension qu'actuellement ; le calcul peut être effectué de deux manières, soit par l'utilisation de l'algèbre linéaire, ce qui produira une valeur précise du coefficient d'atténuation de chaque zone, soit en utilisant la méthode exposée initialement qui suppose une (ou plusieurs) dizaine de balayages pour obtenir une valeur moyenne et un écart type pour chaque point. Il est d'ailleurs possible de procéder à un nombre plus élevé de balayage pour atteindre une précision suffisante pour tout point

b) Définir des zones de dimension plus limitée pour un examen plus fin (par exemple de 1 cm de coté, comportant 100 mini zones par zone, soit une amplification de coefficient de 10, ou se contenter de seulement 25 mini zones par zone)

c) Répartir dans chaque mini zone la valeur obtenue pour chaque zone par exemple en 25 micro zones (voir tableau 3, 4 et 5). Estimer ainsi les valeurs des coefficients d'atténuation de chaque micro zones et les valeurs des bordures

d) Mesurer pas à pas les valeurs de bordures

e) Procéder à un traitement mathématique et numérique fondé sur l'utilisation de la méthode décrite au début de ce texte et montrée dans le tableau 5

f) Procéder éventuellement en deux ou plusieurs étapes, pour les opérations précédentes pour affiner progressivement la définition

g) Procéder à un examen comparatif des valeurs des coefficients d'atténuation estimés par la méthode de répartition proportionnelle et ceux calculés par la méthode exposée. Si une différence importante apparaît ou une micro zone précise ou pour un ensemble de micro zones contiguës, on réduira le nombre de micro zones par zone et l'on procédera à un nouveau calcul de répartition et d'estimation. Si la difficulté persiste on procédera pour l'aire considérée à un calcul des coefficients d'atténuation par les méthodes classiques

h) Editer le document de référence (définition 1 mm) et les documents obtenus par haute définition (définition 20 à 100 microns) et ce dans des présentations nouvelles telles que décrites ou envisagées ci dessus.

Le procédé dont le fonctionnement est ainsi décrit autorise l'obtention d'une définition pouvant être 100 fois plus fine que la résolution actuelle, en utilisant des techniques et des outils de calcul à notre portée.

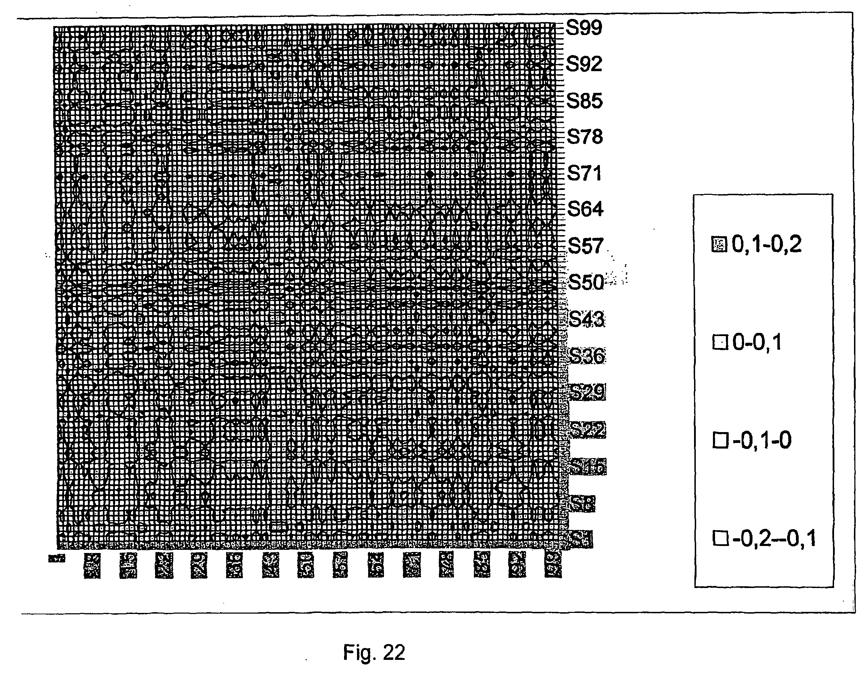

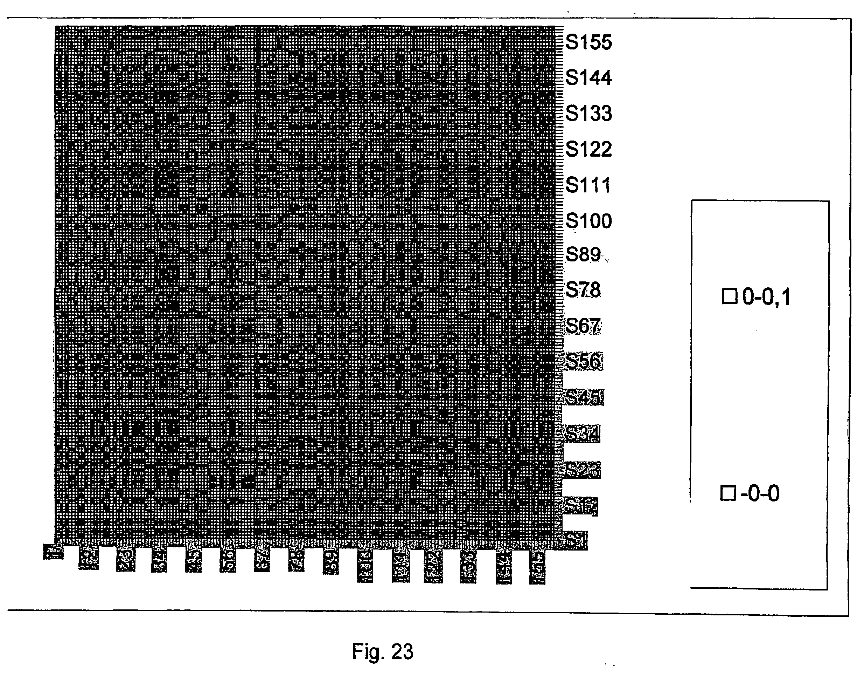

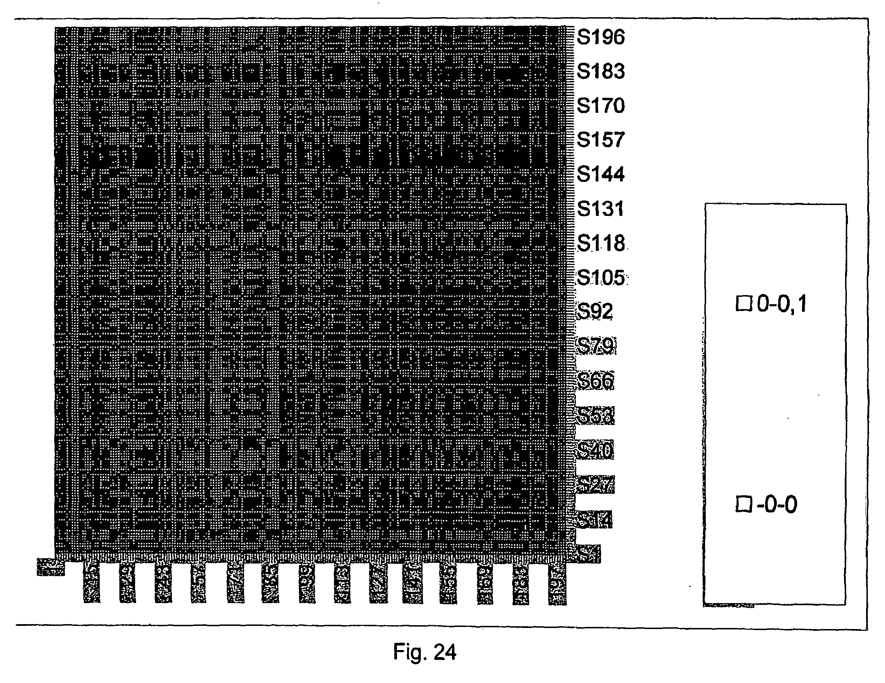

Trois figures 22, 23 et 24 ont été éditées respectivement pour les tableaux 6-2, 6-3 et 6-4 montrant pour une aire réduite des images correspondant aux définitions de 20, 12.5, 10 microns. Dans le dernier cas l'image traduit un processus de calculs relatif à la résolution d'un problème d'algèbre linéaire de 40400 équations et variables, en ne nécessitant que 400 balayages secondaires, plus quatre balayages primaires soit une réduction de 99% de l'irradiation, laquelle irradiation diminue relativement lorsque la dimension de l'aire primaire croît.

Comme ceci a déjà été exprimé l'examen d'une section de 20x20cm avec une définition de 10 microns, détermine 400 millions de points soit autant de profils et nécessite l'inversion d'une matrice 20millex20mille par le procédé classique ; et seulement 80 mille profils soit cinq mille fois moins en deux étapes et un calcul algébrique à notre portée, notamment pour des zones réduites choisies de manière raisonnée ce que le procédé autorise sans aucune complication.

Les progrès techniques en matière de mécanique de déplacement de positionnement de commutation électrique et enfin de calcul permettront sans changer les fondements du système décrit d'atteindre une résolution de un micron ou moins soit la détection de cellules anormales de manière individuelle.

D'ores et déjà on peut utiliser les techniques exposées par l'auteur dans un brevet français N° 00401532, relatif à un moteur piézo électrique à micro crémaillère. Ce procédé consiste à agir par des barrettes piézo électriques à travers une pointe très fine sur un élément mobile dont la surface est striée de manière très fine de sorte à constituer une micro crémaillère. On obtient ainsi des déplacements avec par exemple des pas de 100 microns. Mais on peut aussi utiliser des actuateurs piézo électriques de la société CEDRAT TECHNOLOGIES en France ou NANOMOTION en Israël pour obtenir une série de déplacements avec des pas inférieurs, tout en disposant d'une possibilité d'effort élevé.

A titre d'exemple il est envisageable d'utiliser des actuateurs piézo électriques de CEDRAT TECHNOLOGIES connus sous l'appellation APAs.

Dans le cas présent, il est aussi possible de transmettre un mouvement rotatif grâce à des dispositifs tels que décrits par CEDRAT TECHNOLOGIES, sous l'appellation RPMs.

Les méthodes de traitement des signaux pour obtenir des images numérisées, susceptibles de bénéficier des méthodes de traitement exposées au début peuvent à l'évidence être utilisées dans d'autres domaines que l'imagerie médicale en particulier en ce qui concerne :

- la cartographie des sols, des sous sols, et des pressions atmosphériques

- la photographie et la cinématographie numérique, et la photocopie

- le traitement et la transmission d'images notamment dans le domaine de la télévision

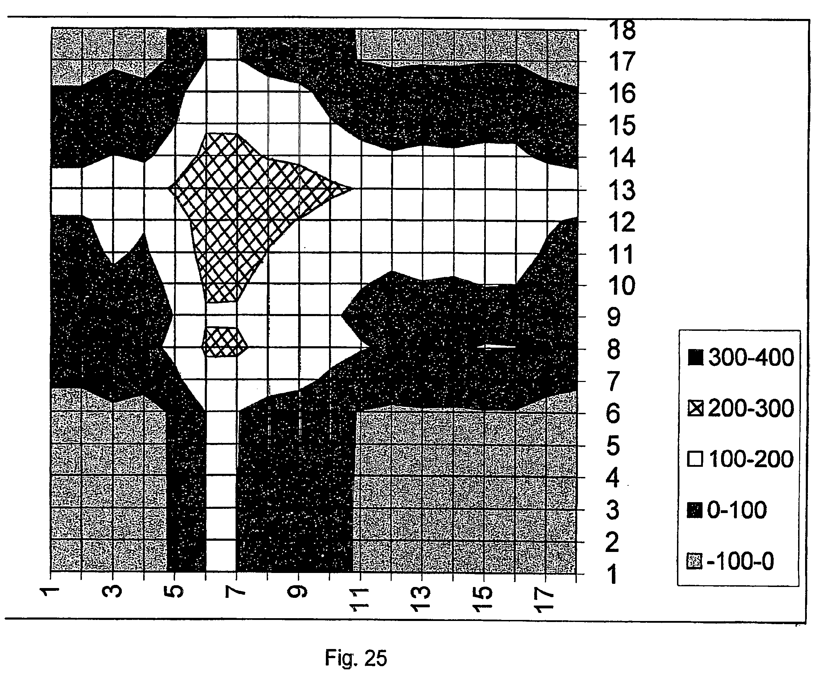

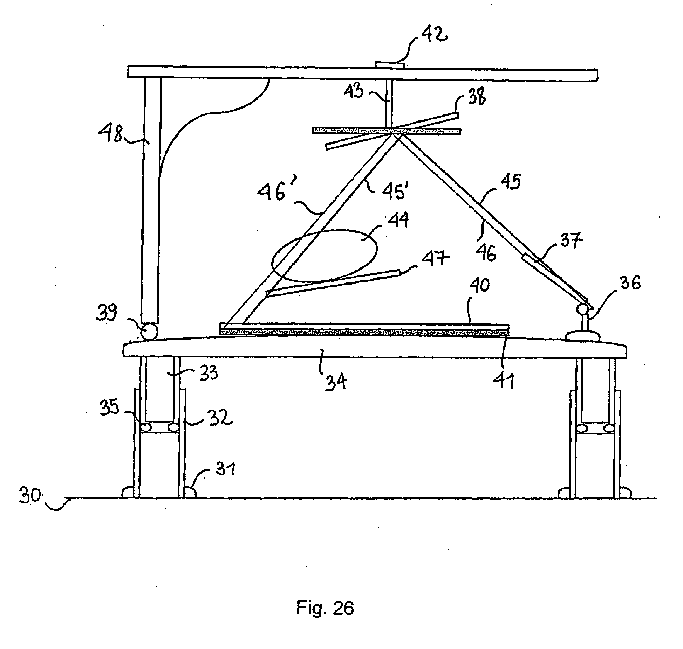

En ce qui concerne l'imagerie médicale obtenue par échographie ultrasonore ou résonance magnétique nucléaire, le procédé d'amélioration de l'image est de même nature, car comme il a été expliqué plus haut la matrice représentative d'une image ou d'une aire déterminée peut être constituée de données associées à des séries de profils centrés sur un ou deux pôles, ou exprimés en coordonnées orthogonales ou obliques. Le procédé d'amplification et ou d'ajustement matriciel conduit de la même manière à une amélioration sensible de la définition.
A titre d'exemple expérimental, la figure 25 montre une image obtenue sur un os de poulet. L'image correspond à un plan de coupe perpendiculaire à la direction longitudinale de l'os. Le quadrillage est gradué en millimètres et les différents niveaux de gris correspondent à différentes valeurs du coefficient d'atténuation de l'os aux rayons X.
Le traitement par laser à infrarouge ou par ultrasons peut aussi être associé aux explorations.
Deux systèmes sont envisagés, différant essentiellement par le mode de déplacement du faisceau laser dans un plan tomographique. Dans les deux cas, on utilisera de préférence un laser YAG NEODYNE de 1064 nanomètres de longueurs d'onde. L'énergie par impulsion est comprise entre 5 à 10 millijoules. Le faisceau est de dimension modifiable par le système optique, pour varier entre 1 et 5 microns. La durée de l'impulsion est comprise entre 1 à 5 nanosecondes. Elle dépendra notamment du temps de réaction des cellules photoélectriques de lecture. Si le temps de réaction des cellules photoélectriques est de l'ordre de la microseconde, il faudra prévoir des temps d'impulsion de même ordre de grandeur et ajuster en conséquence l'énergie du faisceau. Le laser peut peser plusieurs kilogrammes.
On prévoit également d'utiliser une fibre optique déplaçable au voisinage de l'objet à examiner en étant portée par une tige orientable et elle-même déplaçable sur une rampe. Pour des explorations fines, on prévoit d'établir un faisceau plan de fibres optiques, le faisceau étant lui-même déplaçable et orientable.
Un premier système prévoit que pour chaque plan, le laser a une position fixe et le déplacement du faisceau est effectué grâce à un miroir, déplaçable sur une rampe et orientable par rotation en chaque point de sa position sur la rampe. Un deuxième système prévoit que le laser se déplace sur la rampe et soit orientable par rotation en chaque point de la rampe.
La vue du premier système apparaît dans la figure **26**. Sur cette figure, 30 désigne un plancher, 31 un calage des pieds de supports de la table, 32 des cylindres de support, 33 des tubes coulissants, 35 des tores de téflon qui permettent de filtrer les vibrations, 34 une plaque de support réalisée en matériau indéformable en métal rigide, granit ou marbre, pour filtrer ou amortir toute vibration résiduelle, 36 une fixation du laser sur la plaque, 37 un tube laser, 38 un miroir orientable, 39 une fixation de la potence sur le support, 41 une plaque de détection composée de cellules photoélectriques, 42 un rail horizontal de la potence, 40 une plaque de protection des cellules pour éviter la surexposition, 43 une tige de support du miroir, 44 un objet à examiner, 45 ou 46 un rayon laser incident, 45' ou 46' un rayon laser parallèle, 47 une plaque de support de l'objet à examiner, rotative, déplaçable et transparente aux infrarouges et 48 une potence.
Le fonctionnement est le suivant. On place la plaque de support 47 à la hauteur désirée en déplaçant, à l'intérieur du cylindre de support 32, le tube coulissant 33 et une plaquette de support des joints toriques 35. Le tube coulissant 33 n'a pas d'autre contact avec le cylindre de support 32 qu'à travers le joint torique 35 de téflon, ce qui filtre pratiquement toute vibration. On déplace la tige de fixation 43 du miroir 38 jusqu'à la position requise, on oriente le miroir 38 et le laser 37 pour que le rayon laser incident 45 soit orienté comme souhaité.
Différents programmes de balayages permettent l'obtention de profils à basse ou haute définition. Dans le premier cas, la surface élémentaire de chaque cellule est de l'ordre du mm2. Dans le second cas, la taille de la cellule élémentaire peut être de l'ordre de 100 microns et descendre jusqu'au micron (limite pratique compte tenu de la longueur d'onde).
Pour obtenir la définition voulue, on fait passer le faisceau par une optique intégrée au tube laser permettant d'augmenter son diamètre au niveau de l'objet à examiner jusqu'à 1 mm ou de le réduire à un micron.
Dans le cas de faibles diamètres on peut procéder de deux manières, conjointement ou non, en pulsant le fonctionnement pour que la durée d'une impulsion soit réduite de sorte à diminuer les effets thermiques pour l'objet à traverser ou les cellules photoélectriques ou en insérant un dispositif à absorption de l'énergie infrarouge 40.La réalisation d'un plan tomographique s'effectuera donc ainsi :

Dans un premier temps, on réglera le faisceau et l'on ajustera la plaque de détection pour fonctionner par exemple à une définition de un mm et on définira un programme de balayage pour obtenir un nombre de profils égal à la surface en mm2 de la coupe de l'objet et on exécutera le programme de balayage en transférant dans l'ordinateur associé les résultats obtenus sur les cellules.

Dans un deuxième temps, on réglera le faisceau et la plaque de détection pour obtenir la haute définition voulue et on établira un programme de balayage croisé fonction, de la zone à explorer dans la de section de l'objet, de l'angle (généralement droit) entre des deux balayages à haute définition parallèles ou non à haute définition, de l'opacité de l'objet pour régler la durée des impulsions et l'absorption éventuelle.

Les données obtenues au niveau des cellules seront transférées à l'ordinateur qui effectuera le traitement et stockera les résultats.

Dans un troisième temps on utilisera les résultats du calcul pour faire apparaître une image de chaque plan tomographique grâce à un grapheur utilisant ou non des échelles de gris ou des couleurs.

L'utilisation de l'algorithme exposé précédemment s'effectuera dans des conditions identiques. Le procédé ainsi utilisé permet une très considérable économie de temps de calcul dans des proportions identiques à celle obtenue pour le scanner X.

Dans le mode choisi, le miroir 38 pivote autour d'un axe de rotation, de même que le faisceau laser de sorte à obtenir des rayons traversant parallèles 46 et 46'. On pourra déplacer le miroir à une distance suffisante pour obtenir deux séries de profils telles que dans chaque série les rayons traversant soient parallèles entre eux et que les deux séries soient sécantes. un nouveau balayage sous un autre angle, les deux balayages parallèles étant sécants.

On pourra obtenir le volume d'une singularité en comptant tout simplement dans un plan donné les petits carrés ayant un certain niveau de coloration, puis on ajoutant les chiffres obtenus dans les plans voisins et pour des zones adjacentes, entre deux plans limites définis par observation.

La base de données constituée par l'ensemble des résultats relatifs aux différents plans, permettra de mettre en évidence les zones noires résultant de l'opacité absolue de certaines inclusions, qui empêcherait de percevoir certaines zones ainsi cachées.

Pour éclairer cette zone on pourra procéder de deux manières au moins, faire tourner l'objet à examiner pour obtenir plusieurs images sous différents angles après avoir placé des éléments microscopique de repérage pour reconstituer une image complète ou faire tourner la potence 48 pour obtenir des images de zones cachées.

Dans les deux cas, l'objet à examiner devra être placé et éventuellement fixé sur la plaque 47 transparente, susceptible de tourner autour d'un axe.

Dans ce premier système, la réflexion par le miroir devient difficile pour certaines longueurs d'onde, entraînant des pertes d'énergie lumineuse susceptible de varier avec l'angle de réflexion, ce qui peut compliquer le calcul en obligeant à des mesures de référence plus complexes hors la présence de l'objet.

Par contre, la rotation et la translation du miroir 38 est largement facilitée par son faible poids et ne nécessite alors que des actuateurs piézo de faible puissance.

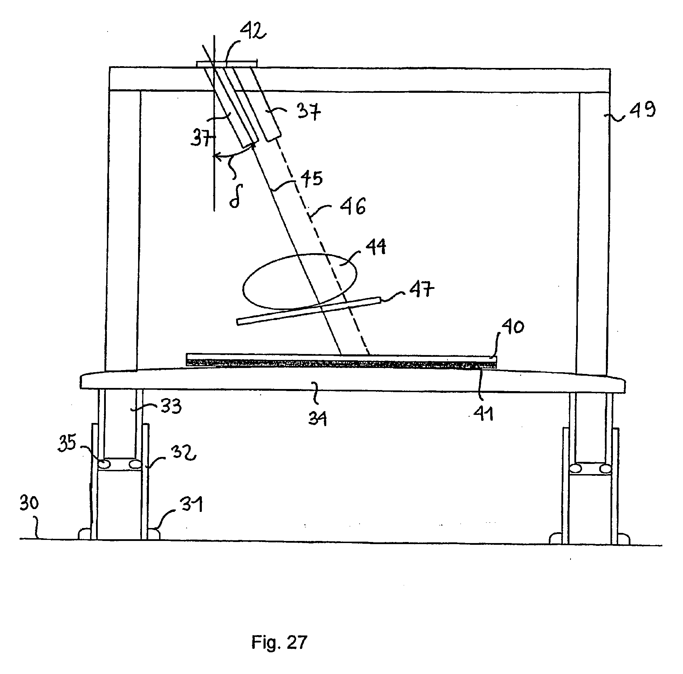

La vue du deuxième système apparaît dans la figure **27**, dans laquelle des références identiques désignent des éléments identiques. La référence 49 désigne un cadre.

Le fonctionnement est le suivant.

On place la plaque de support 34 à la hauteur désirée, en déplaçant à l'intérieur du cylindre de support 32 le tube coulissant 33 une plaquette de support des joints toriques 35. On positionne la plaque de support 47 de l'objet à examiner sous l'angle choisi. Puis on procède de la même manière que pour le premier système. Pour illustrer les balayages on se référera à la figure 18 qui montre le déplacement du faisceau 37, à distance d'une première position et le choix d'une nouvelle inclinaison du faisceau 37 pour obtenir deux balayages sécants. Les faisceaux 45 et 45' sont parallèles entre eux dans chacun des balayages.

Dans ce deuxième système, la translation et la rotation du faisceau laser (37) nécessitent des actuateurs plus puissants. Dans ce cas aussi, l'utilisation de l'algorithme exposé précédemment s'effectuera avec le même avantage. Le procédé ainsi utilisé permet une très considérable économie de temps de calcul dans des proportions identiques à celle obtenues pour le scanner X.

On peut remarquer que l'atténuation peut, dans le cas de l'infrarouge, résulter de plusieurs phénomènes, telles une réflexion à l'intérieur de l'objet, une réfraction dans des zones précises et absorption de l'énergie infrarouge transformée en chaleur.

Il sera donc important, de mesurer aussi l'élévation de la température, dans la mesure du possible, dans différentes zones, pour distinguer les différentes sources d'atténuation.

La nature de l'objet est alors importante et la possibilité de faire tourner l'objet pour différentes mesures, permet l'obtention d'informations utiles à l'évaluation des erreurs résultant de la réflexion ou de la réfraction, lesquelles dépendent de la longueur d'onde et de l'indice de réfraction correspondant pour les différents matériaux.

Pour éviter les phénomènes de réflexion, on prévoit de déposer par évaporation sous vide, une couche mince de matériau de faible indice sur une feuille de matière plastique très mince et d'envelopper l'organisme avec cette feuille 50.

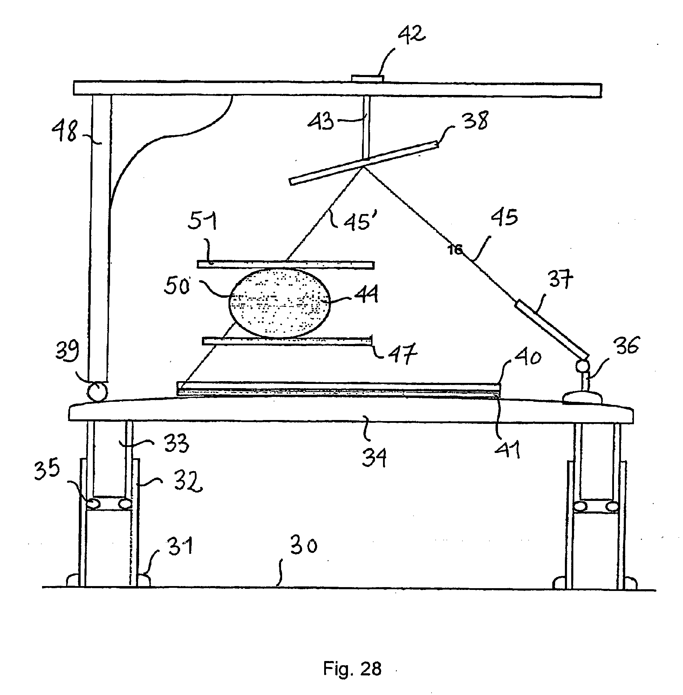

En ce qui concerne la réfraction, dans le cas de l'utilisation d'un laser, c'est à dire de lumière cohérente, le phénomène peut être différent lorsqu'on utilise une lumière non cohérente, mais il existe une certaine réfraction que l'on prévoit de diminuer, figure **28**, en disposant l'organisme ou l'objet 44 entre deux plaques transparentes 47 et 51, l'une des plaques formant la plaque de support décrite dans les exemples illustré par les figures **26** et **27,** revêtues de couches anti-reflets pour éviter la réflexion et rigoureusement parallèles, pour limiter la réfraction qu'elles peuvent provoquer.

Toutefois, une certaine réfraction est possible à l'intérieur du corps 44 qui peut entraîner une déviation du faisceau. On procède alors à l'enveloppement de l'organisme par la feuille plastique 50 ultra mince revêtue d'une couche anti-reflets, à la mise en place des deux plaques transparentes 47 et 51 à la distance nécessaire l'une au dessus de l'autre, à un essai de tarage qui permet de localiser le faisceau sans l'organisme 44 pour différents angles de balayage et à un balayage régulier à travers l'organisme pour mesurer la déviation au niveau de chaque faisceau que l'organisme provoque.

S'il n'y a pas de déviation ou si elle est régulière, l'image obtenue sera d'une qualité acceptable. S'il y a une déviation irrégulière, une correction sera nécessaire pour recadrer l'image, en tenant compte de l'incidence initiale, après rectification régulière, on traitera donc ainsi une matrice dont chaque ligne sera replacée pour régulariser la déviation.

Ce problème disparaît pour les rayons X, pour certaines incidences en infrarouge, ou si le corps traversé comprend des zones dont les indices de réfraction sont voisins. Dans le cas où des différences apparaîtraient, résultant de variations importantes des indices de réfraction, on prévoit de procéder de la façon suivante :

a) on effectue une analyse par scanner X d'une zone déterminée. Cette analyse permet de mettre en évidence des différences de nature du tissu ou des singularités dans la zone analysée.

b) dans cette petite zone, parfaitement repérée, on effectue un balayage par laser infrarouge à très haute définition, et on recueille l'information correspondante, pour dresser une carte de la zone analysée, obtenue par le balayage infrarouge en utilisant un traitement heuristique du parcours de chaque rayon tenant compte d'une évaluation de l'indice de réfraction de chacune des microzones traversées, résultant tant des données obtenues par le scanner X que de la connaissance des coordonnées du point initial et du point final de parcours de la zone.

Ainsi, la combinaison des deux systèmes, scanner X ou scanner à laser infrarouge, autorise une amélioration de la qualité d'image et de sa définition.

Mais dans les deux systèmes de scanner infrarouge ici décrits, le procédé selon l'invention permet de diminuer le nombre de profils à réaliser par laser infrarouge, dans des proportions telles pour les hautes définitions que l'élévation de température restera contrôlable, en évitant ainsi un échauffement anormal de l'objet ou de l'organisme étudié ou si le corps traversé comprend des zones dont les indices de réfraction sont voisins.

Comme ceci a déjà été exposé les temps de calculs resteront dans les limites des moyens normaux de traitement de l'information.

**Revendications**

1. Procédé d'imagerie par exposition d'un corps à des radiations (1), par exemple des rayons X, des faisceaux de lumière infrarouge (45,46), des faisceaux ultrasoniques ou des impulsions magnétiques et traitement du signal en vue de l'obtention d'images représentatives de valeurs associées à chaque point ou zone d'une aire située dans un plan de coupe du corps (20,44) dans lequel, à l'aide d'un dispositif d'imagerie, par exemple d'imagerie médicale, comprenant une source (1,10,37), par exemple à rayons X, et un détecteur (4),

   - on effectue différents profils de balayage croisés dans un plan de coupe du corps,
   - on mesure l'intensité résiduelle de chaque profil de balayage après une traversée du corps et à l'aide d'un ordinateur dûment programmé,

   i) on calcule les valeurs des coefficients d'atténuation, par exemple des rayons X, en autant de points ou zones associés de l'aire du corps (20,44) que des profils de balayage croisés et on construit une image initiale de l'aire du corps à partir d'une matrice initiale des valeurs calculées, **caractérisé en ce que**,

- on effectue une première et une deuxième série de profils de balayage supplémentaires (19,21,42), les profils de balayage de la première série étant croisés par rapport aux profils de balayage de la deuxième série,
- on mesure l'intensité résiduelle de chaque profil de balayage supplémentaire et à l'aide de l'ordinateur dûment programmé,

ii) on construit une matrice rectangulaire amplifiée de la matrice initiale, comportant n lignes et m colonnes de valeurs estimées par interpolation entre deux points des valeurs associées à ces deux points ou par division d'une zone en micro- zones et répartition dans les micro- zones de la valeur associée à cette zone,

iii) on calcule n et m valeurs de bordure de ligne et de colonne de la matrice amplifiée représentative de l'image recherchée, une valeur de bordure de ligne étant la somme des m termes d'une ligne et une valeur de bordure de colonne, la somme des n termes d'une colonne,

iv) on ajuste chacun des n multiplié par m termes de la matrice amplifiée en utilisant un ajustement par une méthode des moindres carrés tenant compte des contraintes de ligne et de colonne relatives aux bordures de la matrice amplifiée, où les n contraintes de ligne sont déterminées par l'intensité résiduelle mesurée pour chaque profil de balayage supplémentaire de la première série et où les m contraintes de colonne sont déterminées par l'intensité résiduelle mesurée pour chaque profil de balayage supplémentaire de la deuxième série, en utilisant la formule suivante :

$$Cij = Bij + \left(\frac{1}{n}\right) * (\rho_j - \sum_{i=1}^{n} Bij) + \left(\frac{1}{m}\right) * (c_i - \sum_{j=1}^{m} Bij) - \left(\frac{1}{nm}\right) * (\sum_{j=1}^{m} \rho_j - \sum_{i}^{n} \sum_{j}^{m} Bij)$$

où, dans cette formule,

Cij = la valeur recherchée
Bij = la valeur estimée initialement
(n) = le nombre de lignes de la matrice rectangle amplifiée
(m) = le nombre de colonnes de la matrice rectangle amplifiée

$$\sum_{i=1}^{n} Cij = \rho_j$$ pour toutes les valeurs de i, la contrainte de la colonne j

$$\sum_{j=1}^{m} Cij = c_i$$ pour toutes les valeurs de j, la contrainte de la ligne i,

de sorte à obtenir les n multiplié par m valeurs ajustées, chacune étant associée à un point de l'image recherchée, par un nombre de profils de balayage supplémentaires limité à n plus m et ainsi réduire l'exposition du corps aux radiations, faisceau à lumière infrarouge, faisceaux ultrasonores ou impulsions magnétiques tout en augmentant la définition de l'image recherchée.

2. Procédé selon la revendication 1, dans lequel on effectue plusieurs fois la première série et la deuxième série de profils de balayage supplémentaires du corps, croisées l'une par rapport à l'autre mais sous des angles différents et on exécute à chaque fois les étapes ii), iii) et iv) pour obtenir une série d'évaluations des valeurs ajustées, chacune associée à un point de l'image recherchée et calculer la moyenne des mesures et estimer un écart type.

3. Dispositif de mise en oeuvre d'un procédé selon la revendication 1 ou 2, comprenant:

- support (2,47) pour recevoir un corps à examiner (44),
- une source (1,10,37) émettant un faisceau de rayons X, un faisceau de lumière infrarouge (45,46), un faisceau ultrasonore ou des impulsions magnétiques sur le corps à examiner (44), et
- un détecteur (4) pour détecter une intensité atténuée en fonction de la traversée des radiations, faisceaux ou impulsions émis par la source à travers le corps à examiner,

pour effectuer différents profils de balayage croisés dans un plan de coupe du corps et mesurer l'intensité résiduelle de chaque profil de balayage après une traversée du corps, et comprenant

- un ordinateur dûment programmé pour :

i) calculer les valeurs des coefficients d'atténuation, par exemple des rayons X, en autant de points ou zones associés d'une aire située dans le plan de coupe du corps (20,44) que des profils de balayage croisés et construire une image initiale de l'aire du corps à partir d'une matrice initiale des valeurs calculées, **caractérisé en ce que** l'ordinateur est dûment programmé pour :

ii) construire une matrice rectangulaire amplifiée de la matrice initiale, comportant n lignes et m colonnes de valeurs estimées par interpolation entre deux points des valeurs associées à ces deux points ou par division d'une zone en micro- zones et répartition dans les micro- zones de la valeur associée à cette zone,

iii) calculer n et m valeurs de bordure de ligne et de colonne de la matrice amplifiée représentative de l'image recherchée, une valeur de bordure de ligne étant la somme des m termes d'une ligne et une valeur de bordure de colonne, la somme des n termes d'une colonne,

iv) ajuster chacun des n multiplié par m termes de la matrice amplifiée en utilisant un ajustement par une méthode des moindres carrés tenant compte des contraintes de ligne et de colonne relatives aux bordures de la matrice amplifiée, où les n contraintes de ligne sont déterminées par l'intensité résiduelle mesurée pour chaque profil de balayage supplémentaire de la première série et où les m contraintes de colonne sont déterminées par l'intensité résiduelle mesurée pour chaque profil de balayage supplémentaire de la deuxième série, en utilisant la formule suivante :

$$Cij = Bij + \left(\frac{1}{n}\right)*(\rho_j - \sum_{i=1}^{n} Bij) + \left(\frac{1}{m}\right)*(c_i - \sum_{j=1}^{m} Bij) - \left(\frac{1}{nm}\right)*(\sum_{j=1}^{m} \rho_j - \sum_{i}^{n} \sum_{j}^{m} Bij)$$

où, dans cette formule,

Cij = la valeur recherchée

Bij = la valeur estimée initialement

(n) = le nombre de lignes de la matrice rectangle amplifiée

(m) = le nombre de colonnes de la matrice rectangle amplifiée

$$\sum_{i=1}^{n} Cij = \rho_j \text{ pour toutes les valeurs de i, la contrainte de la colonne j}$$

$$\sum_{j=1}^{m} Cij = c_i \text{ pour toutes les valeurs de j, la contrainte de la ligne i,}$$

de sorte à obtenir les n multiplié par m valeurs ajustées, chacune étant associée à un point de l'image recherchée, par un nombre de profil de balayage supplémentaires limité à n plus m et ainsi réduire l'exposition du corps aux radiations, faisceau à lumière infrarouge, faisceaux ultrasonores ou impulsions magnétiques tout en augmentant la définition de l'image recherchée.

4. Dispositif selon la revendication 3, **caractérisé en ce qu'**il comprend un guidage par des actuateurs piézoélectriques déplaçant (19,21,42) la source (1,10,37) ou un miroir (38) réfléchissant un faisceau infrarouge émis par la source, pas à pas de manière linéaire pour effectuer la première et la deuxième série des balayages supplémentaires.

5. Dispositif selon la revendication 4, **caractérisé en ce que** le miroir (38) est déplacé en rotation autour d'un pivot, par des actuateurs piézoélectriques, pour réfléchir (45',46') un faisceau laser à infrarouge (45,46) de sorte à obtenir des coefficients relatifs à chaque profil de balayage issus de la première et de la deuxième série de balayages supplémentaires, correspondant à un pas donné du déplacement linéaire du miroir (38).

6. Dispositif selon la revendication 4 ou 5, **caractérisé en ce que** la source (1 ,37) pivote autour d'un axe se déplaçant (19,21,42) lui-même linéairement grâce à des actuateurs piézoélectriques.

7. Dispositif selon la revendication 3, **caractérisé en ce qu'**il comprend des barrettes de détection multiples (4) pour réaliser simultanément plusieurs images parallèles susceptibles d'un traitement individuel ou en trois dimensions.

8. Dispositif selon la revendication 3, **caractérisé en ce qu'**il comprend deux sources perpendiculaires pour effectuer les profils de balayage supplémentaires de la première série perpendiculairement aux profils de balayage supplémentaires de la deuxième série.

9. Dispositif selon l'une des revendications 7 à 8, **caractérisé en ce qu'**il comprend des plaques (19,21) ou autres supports (48,49) de faisceaux, de rayons X ou de lasers, notamment à infrarouge (37) ou d'ultrasons, ou des champs magnétiques sur lesquels les actuateurs piézoélectriques permettent d'orienter ($\alpha,\beta$) les faisceaux ou de les déplacer, en vue d'obtenir grâce à l'ensemble des détecteurs (4,6,40), des informations concernant l'atténuation de chaque profil de balayage de la première et de la deuxième série de balayages supplémentaires, engendré par un pas du faisceau.

10. Dispositif selon l'une des revendications 4 à 9, **caractérisé en ce qu'**il comprend des plaques d'obturation (5,7,22) perforées, lesquelles peuvent être occultées par deux réseaux croisés de fils (12,13) ou lamelles déplaçables pour dégager une obturation (14) ou plusieurs par déplacement des plaques ou lamelles.

11. Dispositif selon la revendication 4 ou 5, **caractérisé en ce qu'**il comprend deux plaques transparentes (47,51), revêtues de couches anti-reflets pour éviter une réflexion du faisceau infrarouge (45') et parallèles pour limiter une réfraction de ce faisceau, le corps à examiner (44) étant disposé entre les deux plaques et en appui contre l'une d'elle servant de plaque de support (47).

12. Ordinateur (27) dûment programmé pour la mise en oeuvre d'un procédé selon la revendication 1.

13. Programme d'ordinateur comportant des instructions pour la mise en oeuvre d'un procédé selon la revendication 1 lorsqu'il est chargé dans un ordinateur (27).

**Patentansprüche**

1. Bildgebendes Verfahren durch Strahlenexposition (1) eines Körpers, zum Beispiel Röntgenstrahlen, Infrarot-Lichtstrahlen (45,46), Ultraschallstrahlen oder magnetischen Impulsen und Signalsverarbeitung, um Bilder zu erhalten, die Werte entsprechen, die mit jedem Punkt oder mit jeder Zone einer Fläche einer Schnittebene des Körpers (20,44) gebunden sind, wodurch, mit Hilfe einer bildgebenden Einrichtung, beispielsweise einer medizinischen bildgebenden Einrichtung, die eine Quelle (1,10,37), zum Beispiel eine Röntgenquelle, und einen Detektor (4) umfasst,

- Verschiedene Scan Profile in einer Schnittebene des Körpers quer durchgeführt werden,
- Eine restliche Intensität für jeden scan Profil gemessen wird, nachdem er durch den Körper durchgedrungen ist, und mit Hilfe eines geeignet programmierten Computers,

i) Werte, beispielsweise Schwächungskoeffizienten der Röntgenstrahlen, für Punkte oder Zonen der Fläche des Körpers (20,44) in einer Anzahl, die gleich den quer durchgeführten Scan Profile ist, berechnet werden und ein erstes Bild der Fläche des Körpers aus einer ersten Matrix der berechneten Werte aufgebaut wird, **dadurch gekennzeichnet, dass**

- Eine erste Reihe und eine zweite Reihe von zusätzlichen Scan Profile (19,21,42) durchgeführt werden, wobei die Scan Profile der ersten Reihe quer in Bezug auf die Scan Profile der zweiten Reihe sind,
- Eine restliche Intensität für jeden zusätzlichen Scan Profil gemessen wird und mit Hilfe des geeignet programmierten Computers,

ii) eine so genannte erweiterte rechteckige Matrix aus der ersten Matrix aufgebaut wird, die n Zeilen und m Spalten von Werten aufweist, die von den Werten, die mit zwei Punkten verbunden sind, durch Interpolation zwischen diesen zwei Punkten, oder durch Dividierung einer Zone in Mikro-Zonen und Verteilung den Wert dieser Zone in den Mikro-Zonen, geschätzt werden,

iii) n und m Zeile und Spalte Randwerten der erweiterten Matrix, die dem gesuchten Bild entspricht, berechnet werden, wobei ein Zeile Randwert, der Summe der m Werten einer Zeile gleich ist und ein Spalte Randwert, der Summe der n Werte einer Spalte gleich ist,

iv) jeder der n mal m Werte der erweiterten Matrix, mit Hilfe einer Methode der kleinsten Quadrate und unter Berücksichtigung der Zeile und Spalte Zwänge in Bezug auf die Randwerten der erweiterten Matrix, wobei die n Zeile Zwänge durch die restliche Intensität bestimmt sind, die für jeden zusätzlichen Scan Profil der ersten Reihe gemessen wird, und wobei die m Spalte Zwänge durch die restliche Intensität bestimmt sind, die für jeden zusätzlichen Scan Profil der zweiten Reihe gemessen wird, und unter Verwendung der folgenden Formel angeglichen wird:

$$C_{ij}=B_{ij}+\frac{1}{n}(\rho_j - \sum_{i=1}^{n} B_{ij})+\frac{1}{m}(c_i - \sum_{j=1}^{m} B_{ij})-\frac{1}{nm}(\sum_{j=1}^{m}\rho_j - \sum_{i=1}^{n}\sum_{j=1}^{m} B_{ij})$$

wobei in dieser Formel
$C_{ij}$ ist der gesuchte Wert,
$B_{ij}$ ist die initiale abgeschätzte Wert
(n) ist die Zahl der Zeilen der initialen Matrix
(m) die Zahl der Spalten der initialen Matrix

$$\sum_{i=1}^{n}C_{ij}=\rho_j \quad \text{für die gesamten Werten i, der Zwang der Spalte j}$$

$$\sum_{j=1}^{m}C_{ij}=c_i \quad \text{für die gesamten Werten j, der Zwang der Zeile i,}$$

so dass die n mal m angeglichenen Werten, die jeweils mit einem Punkt des gesuchten Bild gebunden sind, mittels zusätzlicher Scan Profile in einer zu n plus m beschränkten Anzahl erhalten werden und die Exposition des Körpers gegenüber Strahlung, Infrarot-Lichtstrahlen, Ultraschallstrahlen oder magnetischen Impulsen gemindert wird während die Definition des gesuchte Bildes vergrößert wird.

2. Verfahren nach Anspruch 1, wodurch die erste Reihe und die zweite Reihe der zusätzlichen Scan Profile, eine quer in Bezug auf die andere aber unter verschiedenen Winkeln viermal durchgeführt werden und jedes Mal Schritte ii), iii) und iv) so ausgeführt werden, dass eine Reihe von Schätzungen der angeglichen Werten, die jeweils mit einem Punkt des gesuchten Bild gebunden sind, erhalten werden, einen Mittelwert der Messungen berechnet wird und eine Standardabweichung geschätzt wird.

3. Vorrichtung zur Durchführung eines Verfahrens nach Anspruch 1 oder 2, umfassend:

- Eine Unterstützung (2,47), um einen zu untersuchenden Körper (44) zu unterstützen,
- Eine Quelle (1,10,37), die einen Strahl von Röntgenstrahlen, einen Infrarot-Lichtstrahl (45,46), einen Ultraschallstrahl oder magnetische Impulse auf den zu untersuchenden Körper (44) ausstrahlt, und
- Einen Detektor (4), um eine beim Durchgang der von der Quelle ausgestrahlten Strahlung, Strahlen oder Impulse durch den Körper geschwächte Intensität zu registrieren,

so dass verschiedene Scan Profile in einer Schnittebene des Körpers quer durchgeführt werden und eine restliche Intensität für jeden scan Profil gemessen wird, nachdem er durch den Körper durchgedrungen ist, und

- Ein geeignet programmierten Computer so dass:

i) Werte, beispielsweise Schwächungskoeffizienten der Röntgenstrahlen, für Punkte oder Zonen der Fläche des Körpers (20,44) in einer Anzahl, die gleich der quer durchgeführten Scan Profile ist, berechnet werden und ein erstes Bild der Fläche des Körpers aus einer ersten Matrix der berechneten Werte aufgebaut wird, **dadurch gekennzeichnet, dass**
ii) eine so genannte erweiterte rechteckige Matrix aus der ersten Matrix aufgebaut wird, die n Zeilen und m Spalten von Werten aufweist, die von den Werten, die mit zwei Punkten verbunden sind, durch Interpolation zwischen diesen zwei Punkten, oder durch Dividierung einer Zone in Mikro-Zonen und Verteilung den Wert dieser Zone in den Mikro-Zonen, geschätzt werden,
iii) n und m Zeile und Spalte Randwerten der erweiterten Matrix, die dem gesuchten Bild entspricht, berechnet werden, wobei ein Zeile Randwert, der Summe der m Werten einer Zeile gleich ist und ein Spalte Randwert, der Summe der n Werte einer Spalte gleich ist,

iv) jeder der n mal m Werte der erweiterten Matrix, mit Hilfe einer Methode der kleinsten Quadrate und unter Berücksichtigung der Zeile und Spalte Zwänge in Bezug auf die Randwerten der erweiterten Matrix, wobei die n Zeile Zwänge durch die restliche Intensität bestimmt sind, die für jeden zusätzlichen Scan Profil der ersten Reihe gemessen wird, und wobei die m Spalte Zwänge durch die restliche Intensität bestimmt sind, die für jeden zusätzlichen Scan Profil der zweiten Reihe gemessen wird, und unter Verwendung der folgenden Formel angeglichen wird:

$$C_{ij} = B_{ij} + \frac{1}{n}\left(\rho_j - \sum_{i=1}^{n} B_{ij}\right) + \frac{1}{m}\left(c_i - \sum_{j=1}^{m} B_{ij}\right) - \frac{1}{nm}\left(\sum_{j=1}^{m} \rho_j - \sum_{i=1}^{n} \sum_{j=1}^{m} B_{ij}\right)$$

wobei in dieser Formel
$C_{ij}$ ist der gesuchte Wert,
$B_{ij}$ ist die initiale abgeschätzte Wert
(n) ist die Zahl der Zeilen der initialen Matrix
(m) die Zahl der Spalten der initialen Matrix

$$\sum_{i=1}^{n} C_{ij} = \rho_j$$ für die gesamten Werten i, der Zwang der Spalte j

$$\sum_{j=1}^{m} C_{ij} = c_i$$ für die gesamten Werten j, der Zwang der Zeile i,

so dass die n mal m angeglichenen Werten, die jeweils mit einem Punkt des gesuchten Bild gebunden sind, mittels zusätzlicher Scan Profile in einer zu n plus m beschränkten Anzahl erhalten werden und die Exposition des Körpers gegenüber Strahlung, InfrarotLichtstrahlen, Ultraschallstrahlen oder magnetischen Impulsen gemindert wird während die Definition des gesuchte Bildes vergrößert wird.

4. Vorrichtung nach Anspruch 3, **dadurch gekennzeichnet, dass** sie piezoelektrische Stellglieder (19,21,42) aufweist, die die Quelle (1,10,37) oder einen Spiegel (38), der einen von der Quelle bestrahlten Infrarot-Strahl reflektiert, Schritt für Schritt und auf lineare Weise in Bewegung führt, um die erste Reihe und die zweite Reihe der zusätzlichen Scan Profile durchzuführen.

5. Vorrichtung nach Anspruch 4, **dadurch gekennzeichnet, dass** der Spiegel (38) von piezoelektrischen Stellglieder um einem Drehpunkt gedreht wird, um ein Infrarot-Laserstrahl (45,46) so zu reflektieren (45 ', 46'), dass Koeffizienten für jeden Scan Profil aus der ersten Reihe und der zweiten Reihe der zusätzlichen Scan entsprechend einem bestimmten Schritt der linearen Verschiebung des Spiegels erhalten werden.

6. Vorrichtung nach Anspruch 4 oder 5, **dadurch gekennzeichnet, dass** die Quelle (1,37) um einer Achse dreht, die von piezoelektrischen Stellglieder auf lineare Weise verschieben (19,21,42) wird.

7. Vorrichtung nach Anspruch 3, **dadurch gekennzeichnet, dass** sie vielfache Detektionsstangen (4) umfasst, um gleichzeitig eine Vielzahl von parallelen Bilder zu erstellen, die geeignet sind, einzeln oder in drei Dimensionen verarbeitet zu werden.

8. Vorrichtung nach Anspruch 3, **dadurch gekennzeichnet, dass** sie zwei senkrechte Quellen umfasst, um die zusätzlichen scan Profile der ersten Reihe senkrecht zur zusätzlichen scan Profile der zweiten Reihe durchzufürhen.

9. Vorrichtung nach Anspruch 7 oder 8, **dadurch gekennzeichnet, dass** sie Platten (19,21) oder andere Träger (48,49) für die Röntgen-oder Laserstrahlen, insbesondere Infrarot-Laserstrahlen (37), oder Ultraschall-Strahlen oder magnetische Felder umfasst, auf denen die piezoelektrischen Stellglieder es ermöglichen, die Strahlen so zu orientieren ($\alpha$, $\beta$) oder zu verschieben, dass Informationen in Bezug auf die Schwächung jedes Scan Profils der ersten Reihe und der zweiten Reihe der zusätzlichen Scan Profile, der aus einem Schritt des Strahls erfolgt, mittels der gesamten Detektoren (4,6,40) erhalten werden

10. Vorrichtung nach einem der Ansprüche 4 bis 9, **dadurch gekennzeichnet, dass** sie perforierte Verschlussplatten (5,7,22) aufweist, die durch zwei gekreuzte Netze verschiebbarer Drähte (12,13) oder Streifen so abgeschirmt werden kann, dass die Verschiebung der Platten oder Streifen einen Verschluss befreit.

11. Vorrichtung nach Anspruch 4 oder 5, **dadurch gekennzeichnet, dass** sie zwei transparenten Platten (47,51) umfasst, die mit Anti-Reflexionsschichten beschichtet sind, um eine Reflexion des Infrarot-Strahls (45') zu verhindern, und die parallel sind, um eine Refraktion des Strahls zu begrenzen, wobei der zu untersuchenden Körper (44) zwischen den beiden Platten und anliegend auf einer von ihnen, die als Stützplatte (47) dient, angeordnet ist.

12. Geeignet programmierter Computer (27) zur Durchführung eines Verfahrens nach Anspruch 1.

13. Computerprogramm, das Befehle umfasst, um ein Verfahren nach Anspruch 1 durchzuführen, wenn es in einen Computer (27) geladen wird.


**Claims**

1. Imaging method involving exposing a body to radiation (1), for example X-rays, infra-red light beams (45,46), ultrasound beams or magnetic pulses and processing the signal so as to obtain representative images of values associated with each point or zone of an area of a sectional plane of the body (20,44), in which, with the aid of an imaging device, for example medical imaging device, comprising a source (1,10,37), for example an X-ray source, and a detector (4),

   - crosswise different scan profiles are carried out in a sectional plane of the body,
   - a residual intensity is measured for each scan profile after it has crossed the body and, with the aid of a suitably programmed computer,

      i) values for example of X-rays attenuation coefficients are calculated for the points or zones of the area of the body (20,44) in a number equal to the crosswise scan profiles and an initial image of the area of the body is constructed from an initial matrix of the calculated values,
      **characterised in that**,

         - a first and a second series of supplementary scan profiles (19,21,42) are carried out, the scan profiles of the first series being crossed with respect to the scan profiles of the second series,
         - a residual intensity is measured for each supplementary scan and, with the aid of the suitably programmed computer,

      ii) a so-called expanded rectangular matrix is constructed from the initial matrix, comprising n lines and m columns of values estimated by interpolation, between two points, of the values associated with these two points, or by dividing a zone up into micro-zones and distributing in the micro-zones the value associated with this zone,
      iii) n and m line and column boundary values of the expanded matrix are calculated, representative of the sought image, in which a line boundary value is the sum of the m turns of a line and in which a column boundary value is the sum of the n terms of a column, and
      iv) each of the n times m terms of the expanded matrix is adjusted using a least squares method of adjustment, taking into account the line and column constraints relating to the boundaries of the expanded matrix, in which the n line constraints are determined by the residual intensity of each supplementary scan profile derived from the first scan series, and in which the m column constraints are determined by the residual intensity of each supplementary scan profile derived from the second scan series, and by using the following formula:

$$C_{ij} = B_{ij} + \frac{1}{n}\left(\rho_j - \sum_{i=1}^{n} B_{ij}\right) + \frac{1}{m}\left(c_i - \sum_{j=1}^{m} B_{ij}\right) - \frac{1}{nm}\left(\sum_{j=1}^{m}\rho_j - \sum_{i=1}^{n}\sum_{j=1}^{m} B_{ij}\right)$$

where, in this formula,

$C_{ij}$ is the sought value,
$B_{ij}$ is the initially estimated value
(n) is the number of lines of the rectangle matrix
(m) is the number of columns of the rectangle matrix

$$\sum_{i=1}^{n} C_{ij} = \rho_j \quad \text{for all the values of i, the constraint of the column j}$$

$$\sum_{j=1}^{m} C_{ij} = c_i \quad \text{for all the values of j, the constraint of the line i,}$$

so as to obtain the n times m adjusted values, each associated with a point of the sought image, by means of a number of supplementary scan profiles limited to n plus m and thereby reduce the exposure of the body to radiation, infra-red light beams, ultrasound beams or magnetic pulses, while increasing the definition of the sought image.

2. Method according to claim 1, in which the first series and the second series of the supplementary scan profiles of the body are carried out several times, crossed with respect to one another but under different angles and each time the steps ii), iii) and iv) are carried out so as to obtain a series of evaluations of the value representative of the sought image and to calculate a mean value of the measurements and estimate a standard deviation.

3. Apparatus for implementing a method according to claim 1 or 2, comprising:

   - a support (2,47) for receiving a body to be examined (44),
   - a source (1,10,37) emitting a beam of X-rays, an infra-red light beam (45,46), an ultrasound beam or magnetic pulses onto the body to be examined (44), and
   - a detector (4) for detecting an intensity attenuated according to the passage of the radiation, beams or pulses emitted by the source through the body to be examined,

so that different crossed scan profiles are carried in a sectional plane of the body and the residual intensity is measured for each scan profile after the passage through the body and comprising,

   - a computer suitably programmed to:

   i) calculate the values for example of X-rays attenuation coefficients associated with points or zones of an area of the sectional plane of the body (20,44) in a number equal to said crosswise scan profiles and construct an initial image of said area from an initial matrix of the calculated values,
   **characterised in that** the computer is suitably programmed to :
   ii) construct an expanded rectangular matrix from the initial matrix, comprising n lines and m columns of estimated values by interpolation between two points of the values associated with these two points, or by dividing a zone up into micro-zones and distributing in the micro-zones the value associated with this zone,
   iii) calculate n and m line and column boundary values of the expanded matrix, representative of the sought image, in which a line boundary value is the sum of the m terms of a line and in which a column boundary value is the sum of the n terms of a column and,
   iv) adjust each of the n times m terms of the expanded matrix using a least squares method of adjustment, taking into account the line and column constraints relating to the boundaries of the expanded matrix, in which the n line constraints are determined by the residual intensity measured for each supplementary scan profile of the first series and in which the m column constraints are determined by the residual intensity measured for each supplementary scan profiles of the second series, by using the following formula:

$$C_{ij} = B_{ij} + \frac{1}{n}\left(\rho_j - \sum_{i=1}^{n} B_{ij}\right) + \frac{1}{m}\left(c_i - \sum_{j=1}^{m} B_{ij}\right) - \frac{1}{nm}\left(\sum_{j=1}^{m} \rho_j - \sum_{i=1}^{n}\sum_{j=1}^{m} B_{ij}\right)$$

where, in this formula,

$C_{ij}$ is the sought value,
$B_{ij}$ is the initially estimated value
(n) is the number of lines of the rectangle matrix
(m) is the number of columns of the rectangle matrix

$$\sum_{i=1}^{n} C_{ij} = \rho_j \quad \text{for all the values of i, the constraint of the column j}$$

$$\sum_{j=1}^{m} C_{ij} = c_i \quad \text{for all the values of j, the constraint of the line i,}$$

so as to obtain the n times m adjusted values, each associated with a point of the sought image, by means of a number of supplementary scan profiles limited to n plus m and thereby reduce the exposure of the body to radiation, infra-red light beams, ultrasound beams or magnetic pulses, while increasing the definition of the sought image.

4. Apparatus according to claim 3, **characterised in that** it comprises a guidance by means of piezoelectric actuators (19,21,42) displacing the source (1,10,37) or a mirror (38) reflecting an infra-red beam emitted by the source, step by step in a linear manner so as to carry out the first and the second series of the supplementary scan profiles.

5. Apparatus according to claim 4, **characterised in that** the mirror (38) is displaced by rotation about a pivotal point, by means of piezoelectric actuators, to reflect (45',46') an infra-red laser beam (45,46) so as to obtain coefficients relating to each scan profile derived from the first and the second series of supplementary scans, corresponding to a given step of the linear displacement of the mirror.

6. Apparatus according to claim 4 or 5, **characterised in that** the source (1,37) pivots about an axis that itself moves (19,21,42) linearly by means of piezoelectric actuators.

7. Apparatus according to claim 3, **characterised in that** it comprises multiple detection bars (4) for producing simultaneously a plurality of parallel images capable of individual processing or processing in three dimensions.

8. Apparatus according to claim 3, **characterised in that** it comprises two perpendicular sources in order to carry out the supplementary scan profiles of the first series perpendicular to the supplementary scan profiles of the second series.

9. Apparatus according to claim 7 or 8, **characterised in that** it comprises plates (19,21) or other supports (48,49) for X-ray or laser beams, in particular infra-red laser beams (37) or ultrasound beams or magnetic fields, on which the piezoelectric actuators enable the beams to be oriented ($\alpha,\beta$) or to be displaced, so as to obtain by means of the array of the detectors (4,6,40), information relating to the attenuation of each scan profile of the first and second supplementary scan series, produced by a step of the beam.

10. Apparatus according to one of the claims 4 to 9, **characterised in that** it comprises perforated blocking plates (5,7,22) which may be shielded by two crossed networks of displaceable wires (12,13) or strips so as to effect one or more unblockings by displacement of the plates or strips.

11. Apparatus according to claim 4 or 5, **characterised in that** it comprises two transparent plates (47,51) coated with anti-reflecting layers to prevent a reflection of the infra-red beam (45') and which are parallel in order to limit a refraction of this beam, the body to be examined (44) being arranged between the two plaques and resting against one of them serving as support plate (47).

12. Suitably programmed computer (27) for carrying out a method according to claim 1.

13. Computer program comprising instructions for carrying out a method according to claim 1 when it is loaded in a computer (27).

Fig. 1

Fig. 2

EP 1 756 774 B1

Fig. 3

Fig. 4     Fig. 5

Fig. 6      Fig. 7          Fig. 8

31

Fig. 9

Fig. 10

Fig. 11

Fig. 12

13

Fig. 13

EP 1 756 774 B1

Fig. 14

Fig. 15

Fig. 16

Fig. 17

| | 1 | 2 | 3 | 4 | 5 | 6 | 7 | 8 | 9 | 10 | 11 | 12 | 13 | 14 | 15 | 16 | 17 | 18 | 19 | 20 | 21 |
|---|---|---|---|---|---|---|---|---|---|---|---|---|---|---|---|---|---|---|---|---|---|
| 1 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,64 | 0,64 | 0,64 | 0,6 | 0,6 | 16 |
| 2 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,64 | 0,64 | 0,64 | 0,6 | 0,6 | 16 |
| 3 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,64 | 0,64 | 0,64 | 0,6 | 0,6 | 16 |
| 4 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,64 | 0,64 | 0,64 | 0,6 | 0,6 | 16 |
| 5 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,64 | 0,64 | 0,64 | 0,6 | 0,6 | 16 |
| 6 | 0,88 | 0,88 | 0,96 | 0,96 | 0,96 | 0,96 | 0,96 | 0,96 | 0,96 | 0,96 | 0,72 | 0,72 | 0,72 | 0,72 | 0,72 | 0,64 | 0,64 | 0,64 | 0,6 | 0,6 | 16 |
| 7 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 |
| 8 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 |
| 9 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 |
| 10 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 |
| 11 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,8 | 0,8 | 0,8 | 0,8 | 0,8 | 16,8 |
| 12 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 1 | 1 | 18,4 |
| 13 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 1 | 1 | 18,4 |
| 14 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 1 | 1 | 18,4 |
| 15 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 1 | 1 | 18,4 |
| 16 | 14,4 | 14,4 | 14,4 | 14,4 | 14,4 | 13,2 | 13,2 | 13,2 | 13,2 | 13,2 | 11,6 | 11,6 | 11,6 | 11,6 | 11,6 | 12 | 12 | 12 | 12 | 12 | 256 |
| 17 | | | | | | | | | | | | | | | | | | | | | |
| 18 | | | | | | | | | | | | | | | | | | | | | |

Fig. 18

EP 1 756 774 B1

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 |
|---|---|---|---|---|---|---|---|---|---|---|---|---|---|---|---|---|---|---|---|---|---|---|
| 1 | 0,88 | 0,88 | 0,88 | 0,88 | 0,88 | 1 | 0,96 | 0,96 | 0,96 | 0,96 | 0,7 | 0,7 | 0,72 | 0,7 | 0,7 | 0,64 | 0,64 | 0,64 | 0,64 | 0,64 | 16 | 18 |
| 2 | 0,88 | 0,88 | 0,88 | 0,88 | 0,88 | 1 | 0,96 | 0,96 | 0,96 | 0,96 | 0,7 | 0,7 | 0,72 | 0,7 | 0,7 | 0,64 | 0,64 | 0,64 | 0,64 | 0,64 | 16 | 14 |
| 3 | 0,88 | 0,88 | 0,88 | 0,88 | 0,88 | 1 | 0,96 | 0,96 | 0,96 | 0,96 | 0,7 | 0,7 | 0,72 | 0,7 | 0,7 | 0,64 | 0,64 | 0,64 | 0,64 | 0,64 | 16 | 14 |
| 4 | 0,88 | 0,88 | 0,88 | 0,88 | 0,88 | 1 | 0,96 | 0,96 | 0,96 | 0,98 | 0,7 | 0,7 | 0,72 | 0,7 | 0,7 | 0,64 | 0,64 | 0,64 | 0,64 | 0,64 | 16 | 14 |
| 5 | 0,88 | 0,88 | 0,88 | 0,88 | 0,88 | 1 | 0,96 | 0,96 | 0,96 | 0,96 | 0,7 | 0,7 | 0,72 | 0,7 | 0,7 | 0,64 | 0,64 | 0,64 | 0,64 | 0,64 | 16 | 16 |
| 6 | 0,96 | 0,96 | 0,96 | 0,96 | 0,96 | 0,9 | 0,88 | 0,88 | 0,88 | 0,88 | 0,7 | 0,7 | 0,72 | 0,7 | 0,7 | 0,8 | 0,8 | 0,8 | 0,8 | 0,8 | 16,8 | 18,8 |
| 7 | 0,96 | 0,96 | 0,96 | 0,96 | 0,96 | 0,9 | 0,88 | 0,88 | 0,88 | 0,88 | 0,7 | 0,7 | 0,72 | 0,7 | 0,7 | 0,8 | 0,8 | 0,8 | 0,8 | 0,8 | 16,8 | 14,8 |
| 8 | 0,96 | 0,96 | 0,96 | 0,96 | 0,96 | 0,9 | 0,88 | 0,88 | 0,88 | 0,88 | 0,7 | 0,7 | 0,72 | 0,7 | 0,7 | 0,8 | 0,8 | 0,8 | 0,8 | 0,8 | 16,8 | 14,8 |
| 9 | 0,96 | 0,96 | 0,96 | 0,96 | 0,96 | 0,9 | 0,88 | 0,88 | 0,88 | 0,88 | 0,7 | 0,7 | 0,72 | 0,7 | 0,7 | 0,8 | 0,8 | 0,8 | 0,8 | 0,8 | 16,8 | 18,8 |
| 10 | 0,96 | 0,96 | 0,96 | 0,96 | 0,96 | 0,9 | 0,88 | 0,88 | 0,88 | 0,88 | 0,7 | 0,7 | 0,72 | 0,7 | 0,7 | 0,8 | 0,8 | 0,8 | 0,8 | 0,8 | 16,8 | 16,8 |
| 11 | 1,04 | 1,04 | 1,04 | 1,04 | 1,04 | 0,8 | 0,8 | 0,8 | 0,8 | 0,8 | 0,9 | 0,9 | 0,88 | 0,9 | 0,9 | 0,96 | 0,96 | 0,96 | 0,96 | 0,96 | 18,4 | 20,4 |
| 12 | 1,04 | 1,04 | 1,04 | 1,04 | 1,04 | 0,8 | 0,8 | 0,8 | 0,8 | 0,8 | 0,9 | 0,9 | 0,88 | 0,9 | 0,9 | 0,96 | 0,96 | 0,98 | 0,96 | 0,96 | 18,4 | 16,4 |
| 13 | 1,04 | 1,04 | 1,04 | 1,04 | 1,04 | 0,8 | 0,8 | 0,8 | 0,8 | 0,8 | 0,9 | 0,9 | 0,88 | 0,9 | 0,9 | 0,96 | 0,96 | 0,96 | 0,96 | 0,96 | 18,4 | 16,4 |
| 14 | 1,04 | 1,04 | 1,04 | 1,04 | 1,04 | 0,8 | 0,8 | 0,8 | 0,8 | 0,8 | 0,9 | 0,9 | 0,88 | 0,9 | 0,9 | 0,96 | 0,96 | 0,96 | 0,96 | 0,96 | 18,4 | 20,4 |
| 15 | 1,04 | 1,04 | 1,04 | 1,04 | 1,04 | 0,8 | 0,8 | 0,8 | 0,8 | 0,8 | 0,9 | 0,9 | 0,88 | 0,9 | 0,9 | 0,96 | 0,96 | 0,96 | 0,96 | 0,98 | 18,4 | 18,4 |
| 16 | 14,4 | 14,4 | 14,4 | 14,4 | 14,4 | 13 | 13,2 | 13,2 | 13,2 | 13,2 | 12 | 12 | 11,6 | 12 | 12 | 12 | 12 | 12 | 12 | 12 | 256 | 256 |
| 17 | 13,6 | 14,4 | 13,8 | 13,6 | 15,2 | 13 | 14 | 13,6 | 12,8 | 12,6 | 12 | 13 | 12,4 | 11 | 11 | 11,4 | 12 | 12,4 | 12,8 | 11,6 | | |
| 18 | 13,6 | 28 | 41,8 | 55,4 | 70,6 | 84 | 97,6 | 111 | 124 | 137 | 148 | 161 | 173 | 184 | 196 | 207 | 219 | 231,4 | 244,2 | 255,8 | | |
| 19 | 14,4 | 28,8 | 43,2 | 57,6 | 72 | 85 | 98,4 | 112 | 125 | 138 | 150 | 161 | 173 | 184 | 196 | 208 | 220 | 232 | 244 | 256 | | |

Fig. 19

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 |
|---|---|---|---|---|---|---|---|---|---|---|---|---|---|---|---|---|---|---|---|---|---|---|---|
| | 0,93 | 0,98 | 0,94 | 0,93 | 1,033 | 1,047 | 1,11 | 1,09 | 1,033 | 1,02 | 0,82 | 0,89 | 0,873 | 0,78 | 0,8067 | 0,7 | 0,74 | 0,7667 | 0,793 | 0,713 | 17,887 | 17,99 | |
| 1 | 0,88 | 0,88 | 0,88 | 0,88 | 0,88 | 0,96 | 0,96 | 0,96 | 0,88 | 0,96 | 0,72 | 0,72 | 0,72 | 0,72 | 0,72 | 0,64 | 0,64 | 0,64 | 0,64 | 0,64 | 16 | 18 | 2 |
| | 0,73 | 0,78 | 0,74 | 0,73 | 0,833 | 0,847 | 0,91 | 0,89 | 0,833 | 0,82 | 0,62 | 0,69 | 0,673 | 0,58 | 0,6067 | 0,5 | 0,54 | 0,5667 | 0,593 | 0,513 | 13,987 | | |
| 2 | 0,88 | 0,88 | 0,88 | 0,88 | 0,88 | 0,96 | 0,96 | 0,96 | 0,98 | 0,96 | 0,72 | 0,72 | 0,72 | 0,72 | 0,72 | 0,64 | 0,64 | 0,64 | 0,64 | 0,64 | 16 | 14 | -2 |
| | 0,73 | 0,78 | 0,74 | 0,73 | 0,833 | 0,847 | 0,91 | 0,89 | 0,833 | 0,82 | 0,62 | 0,69 | 0,673 | 0,58 | 0,6067 | 0,5 | 0,54 | 0,5667 | 0,593 | 0,513 | 13,987 | | |
| 3 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,84 | 0,64 | 0,64 | 0,64 | 0,64 | 16 | 14 | -2 |
| | 0,93 | 0,98 | 0,94 | 0,93 | 1,033 | 1,047 | 1,11 | 1,09 | 1,033 | 1,02 | 0,82 | 0,89 | 0,873 | 0,78 | 0,8067 | 0,7 | 0,74 | 0,7667 | 0,793 | 0,713 | 17,987 | | |
| 4 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,64 | 0,64 | 0,64 | 0,64 | 0,64 | 16 | 18 | 2 |
| | 0,83 | 0,88 | 0,84 | 0,83 | 0,933 | 0,947 | 1,01 | 0,99 | 0,933 | 0,92 | 0,72 | 0,79 | 0,773 | 0,68 | 0,7087 | 0,6 | 0,64 | 0,6667 | 0,693 | 0,613 | 15,987 | | |
| 5 | 0,88 | 0,88 | 0,88 | 0,88 | 0,88 | 0,96 | 0,96 | 0,96 | 0,96 | 0,96 | 0,72 | 0,72 | 0,72 | 0,72 | 0,72 | 0,64 | 0,64 | 0,64 | 0,64 | 0,64 | 16 | 16 | 0 |
| | 1,01 | 1,06 | 1,02 | 1,01 | 1,113 | 0,967 | 1,03 | 1,01 | 0,953 | 0,94 | 0,82 | 0,89 | 0,873 | 0,78 | 0,8067 | 0,86 | 0,9 | 0,9267 | 0,953 | 0,873 | 18,787 | | |
| 6 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 | 18,8 | 2 |
| | 0,81 | 0,86 | 0,82 | 0,81 | 0,913 | 0,767 | 0,83 | 0,81 | 0,753 | 0,74 | 0,62 | 0,69 | 0,673 | 0,58 | 0,6067 | 0,66 | 0,7 | 0,7267 | 0,753 | 0,673 | 14,787 | | |
| 7 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 | 14,8 | -2 |
| | 0,81 | 0,86 | 0,82 | 0,81 | 0,913 | 0,767 | 0,83 | 0,81 | 0,763 | 0,74 | 0,62 | 0,69 | 0,673 | 0,58 | 0,6067 | 0,66 | 0,7 | 0,7267 | 0,763 | 0,673 | 14,787 | | |
| 8 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 | 14,8 | -2 |
| | 1,01 | 1,06 | 1,02 | 1,01 | 1,113 | 0,967 | 1,03 | 1,01 | 0,953 | 0,94 | 0,82 | 0,89 | 0,873 | 0,78 | 0,8067 | 0,86 | 0,9 | 0,9267 | 0,953 | 0,873 | 18,787 | | |
| 9 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 | 18,8 | 2 |
| | 0,91 | 0,96 | 0,92 | 0,91 | 1,013 | 0,867 | 0,93 | 0,91 | 0,853 | 0,84 | 0,72 | 0,79 | 0,773 | 0,68 | 0,7067 | 0,76 | 0,8 | 0,8267 | 0,853 | 0,773 | 16,787 | | |
| 10 | 0,96 | 0,96 | 0,96 | 0,96 | 0,96 | 0,88 | 0,88 | 0,88 | 0,88 | 0,88 | 0,72 | 0,72 | 0,72 | 0,72 | 0,72 | 0,8 | 0,8 | 0,8 | 0,8 | 0,8 | 16,8 | 16,8 | 0 |
| | 1,09 | 1,14 | 1,1 | 1,09 | 1,193 | 0,887 | 0,95 | 0,93 | 0,873 | 0,86 | 0,98 | 1,05 | 1,033 | 0,94 | 0,9667 | 1,02 | 1,06 | 1,0867 | 1,113 | 1,033 | 20,387 | | |
| 11 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 0,96 | 0,96 | 18,4 | 20,4 | 2 |
| | 0,89 | 0,94 | 0,9 | 0,89 | 0,993 | 0,687 | 0,75 | 0,73 | 0,673 | 0,66 | 0,78 | 0,85 | 0,833 | 0,74 | 0,7667 | 0,82 | 0,86 | 0,8867 | 0,913 | 0,833 | 16,387 | | |
| 12 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 0,96 | 0,96 | 18,4 | 16,4 | -2 |
| | 0,89 | 0,94 | 0,9 | 0,89 | 0,993 | 0,687 | 0,75 | 0,73 | 0,673 | 0,66 | 0,78 | 0,85 | 0,833 | 0,74 | 0,7667 | 0,82 | 0,86 | 0,8867 | 0,913 | 0,833 | 16,387 | | |
| 13 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 0,96 | 0,96 | 18,4 | 16,4 | -2 |
| | 1,09 | 1,14 | 1,1 | 1,09 | 1,193 | 0,887 | 0,95 | 0,93 | 0,873 | 0,86 | 0,98 | 1,05 | 1,033 | 0,94 | 0,9667 | 1,02 | 1,06 | 1,0867 | 1,113 | 1,033 | 20,387 | | |
| 14 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 0,96 | 0,96 | 18,4 | 20,4 | 2 |
| | 0,99 | 1,04 | 1 | 0,99 | 1,093 | 0,787 | 0,85 | 0,83 | 0,773 | 0,76 | 0,88 | 0,95 | 0,933 | 0,84 | 0,8867 | 0,92 | 0,86 | 0,9867 | 1,013 | 0,933 | 18,387 | | |
| 15 | 1,04 | 1,04 | 1,04 | 1,04 | 1,04 | 0,8 | 0,8 | 0,8 | 0,8 | 0,8 | 0,88 | 0,88 | 0,88 | 0,88 | 0,88 | 0,96 | 0,96 | 0,96 | 0,96 | 0,96 | 18,4 | 18,4 | 0 |
| 16 | 14,4 | 14,4 | 14,4 | 14,4 | 14,4 | 13,2 | 13,2 | 13,2 | 13,2 | 13,2 | 11,6 | 11,6 | 11,6 | 11,6 | 11,6 | 12 | 12 | 12 | 12 | 12 | 256 | 256 | |
| 17 | 13,8 | 14,4 | 13,8 | 13,6 | 15,2 | 13 | 14 | 13,6 | 12,8 | 12,6 | 11,6 | 12,6 | 12,4 | 11 | 11,4 | 11,4 | 12 | 12,4 | 12,8 | 11,6 | 255,8 | | |
| 18 | | | | | | | | | | | | | | | | | | | | | | | |
| 19 | -0,8 | 0 | -0,6 | -0,8 | 0,8 | -0,2 | 0,8 | 0,4 | -0,4 | -0,6 | 0 | 1 | 0,8 | -0,6 | -0,2 | -0,6 | 0 | 0,4 | 0,8 | -0,4 | -0,2 | | |
| | -0,05 | 0 | -0,04 | -0,05 | 0,053 | -0,01 | 0,05 | 0,03 | -0,03 | -0,04 | 0 | 0,07 | 0,053 | -0 | -0,013 | -0,04 | 0 | 0,0267 | 0,053 | -0,03 | | | |

Fig. 20

Fig. 21

EP 1 756 774 B1

Fig. 22

Fig. 23

Fig. 24

Fig. 25

Fig. 26

Fig. 27

Fig. 28

**EP 1 756 774 B1**

## RÉFÉRENCES CITÉES DANS LA DESCRIPTION

**Documents brevets cités dans la description**

- US 3924131 A
- US 3919552 A
- US 5241471 A
- EP 99401358 A
- FR 00401532